# EUROPEAN PATENT SPECIFICATION

(11) **EP 4 221 895 B1**
(45) Date of publication and mention of the grant of the patent: **16.09.2026**
(21) Application number: 21876625.1
(22) Date of filing: 01.10.2021
(51) Int. Cl.: B01L 3/14, B01L 3/00, B01L 3/02, B01L 3/12, G01N 1/38, A61B 10/00

(54) **BIOFLUID SELF-COLLECTION AND PROCESSING DEVICE**
BIOFLUID-SELBSTSAMMEL- UND VERARBEITUNGSVORRICHTUNG
DISPOSITIF D'AUTO-COLLECTE DE TRAITEMENT DE LIQUIDE BIOLOGIQUE

(30) Priority: 02.10.2020 US 202063086929 P
(43) Date of publication of application: 09.08.2023
(73) Proprietor: Arizona Board of Regents on behalf of the University of Arizona, Tucson, Arizona 85701 (US); Wren Laboratories, LLC, Branford, Connecticut 06405 (US); Costa Devices Limited, Tortola (VG)
(72) Inventor: ZENHAUSERN, Frederic, Phoenix, Arizona 85004 (US); BARRETT, Matthew, Phoenix, Arizona 85004 (US); THOMAS, Baiju, Phoenix, Arizona 85004 (US); NORDQUIST, Alan, Phoenix, Arizona 85004 (US); LACOMBE, Jerome, Phoenix, Arizona 85004 (US); MODLIN, Irvin, Branford, Connecticut 06405 (US); KIDD, Mark, Branford, Arizona 06405 (US)
(74) Representative: Schmidt, Christian
(86) International application number: PCT/US2021/053233
(87) International publication number: WO 2022/072876

(56) References cited:
- JP-A- 2016 211 890
- US-A1- 2004 014 203
- US-A1- 2005 096 563
- US-A1- 2008 017 577
- US-A1- 2010 093 551
- US-A1- 2013 092 690
- US-B2- 9 113 850
- US-B2- 9 816 087

## Description

### BACKGROUND OF THE INVENTION

The devices and methods provided herein address a need in the art to reliably self-collect fluid samples by an individual user for subsequent testing, such as by shipping the collected specimen to a laboratory.

Although there are diagnostics companies providing consumer or at home testing kits involving blood or saliva collection devices, those devices generally require an individual to deposit a specimen (e.g. saliva spit) into a collection tube containing some stabilization buffer solution or the like. There is a risk of the user aspirating the liquid from the tube during the collection or being exposed to hazardous volatiles from the buffer solution. For these reasons, FDA guidelines require isolating the steps of saliva collection from the transfer to the buffer solution and collection tube so that the individual is safely protected. Such a procedure, however, must preserve the integrity of the specimen for further molecular testing. As one example, U.S. Pat. No. 9,732,376 describes a sample collection device for collecting a saliva sample for subsequent transport to a laboratory for DNA analysis. That device, however, suffers from a number of disadvantages, including lack of an integrated reservoir for holding buffer, ability to reliably cut into the reservoir with an insert that fits into off-the-shelf commercially available tubes for holding samples. Those devices are also not compatible of integration with a means of processing of the sample, including for direct testing of a marker on a fluid sample.

From the forgoing, there is a need in the art for self-collection devices that can collect a sample and hold a reagent volume that is to be introduced to the sample, and/or that can process the sample in an integrated, reliable and safe manner.
US 2013/0092690 A1 discloses a seal cap assembly for use with a biological specimen container, the cap assembly including an integral ampoule that holds a pre-filled agent. A piercer is movably disposed in the cap assembly to pierce into the ampoule. When the cap assembly is attached to a biological specimen container, the ampoule is pierced, the pre-filled agent in the ampoule is mixed with the specimen, and the seal cap assembly seals the biological specimen container. The seal cap assembly, together with a biological specimen container, permits stabilization, storage, and transport of biological fluid samples.
JP 2016-211890 relates to a saliva sampling container set assembly for DNA analysis, which is configured to facilitate a process up to attaching a cap on a container body and prevent spilling of liquid accommodated therein.
US 2004/0014203 A1 discloses a sample testing device. The sample testing device has a buffer container that can hold buffer fluid, a filter with a securement for holding a test strip, the test strip, a test strip container having a receptacle to accommodate the filter, so that when the filter is held therein the test strip is disposed in the receptacle, and a sample collector for holding a sample. The sample collector receives the buffer container, and the sample collector has a piercing member which, when the buffer container is placed in the sample collector, pierces the buffer container. Buffer fluid in the buffer container then contacts the sample. As buffer fluid flows through the sample collector, the buffer fluid that has contacted the sample passes through the filter to the test strip.

### BRIEF SUMMARY OF THE INVENTION

The present invention provides a kit for self-collection and processing of a biofluid that may contain a biological specimen or a biomarker of the biological specimen from a user as defined by claim 1. Embodiments of the invention are defined by the dependent claims. For better understanding of the invention, the present disclosure provides further examples, which relate to a method for self-collecting and processing a biofluid. These methods do not form part of the present invention.

The invention provides a reliable, efficient and safe platform for self-collection of biofluids (e.g., biofluids that may or may not contain a biological specimen and/or a biomarker thereof), mixing of a reagent and a fluid, and related sample collection, processing and fluid mixing methods. As described, the sample collection can include collection of saliva. The processing aspect is used broadly herein and may refer to initial sample manipulation, including cell lysis, extraction, isolation and/or stabilization. Processing may also refer to a detection assay, including to detect one or more markers in a sample, such as biomarkers associated with a disease or infection in saliva. Accordingly, processing may include use of biomarkers or antibodies in an array configuration for detecting protein or viral particles associated with a pathogen, such as in a COVID test.

The present invention addresses the need in the art for a safe self-collection device that is self-integrated with all the necessary reagents for use with a self-collected sample. Relevant components include: (1) a mouthpiece for receiving a biofluid specimen; (2) a collection tube; (3) a tube insert with features that provide cutting or piercing a sealing membrane that forms a reagent reservoir; (4) a hollow cap with a cavity to contain some liquid, solid or gas reagents and (5) a sealing membrane to close the cavity of the cap. Typically, a user will spit less than 1 ml of saliva using a mouthpiece into the collection tube equipped with the tube insert. The mouthpiece is removed and replaced by the cap which can screw onto or into the collection tube, leading to the piercing of the reservoir membrane material by the tube insert piercing end. The piercing of the reservoir membrane, along with the specially configured tube insert, allows the reagents to mix with the biofluid specimen, such as the saliva. The device is compatible with any of a desired ratio range of reagent and specimen volume, such as an about 2:1 ratio.

This device can, therefore, preserve the biological specimen and inactivate pathogens such as viral or bacterial organisms while stabilizing the nucleic acid (e.g. RNA or DNA) for further molecular analysis. These analyses can comprise omics such as gene expression, proteomics, metabolomics or standard chemistries.

The devices are also compatible with substantially real-time assays. As described, the biofluid can be collected and "processed" with the reagent volume. The membrane may then be swapped out with a biomarker detection membrane that is porous to the mixed reagent and biofluid. Inverting the collection tube can force the mixed solution through the biomarker membrane and desired biomarkers in the biofluid detected. For example, the detection may be by optical detection of a colorimetric change. In this manner, any of the devices and methods may be described as providing real-time detection of a biomarker. This can significantly improve diagnostic times by avoiding having to transport the collection device to a lab for sample analysis.

Provided herein are devices for self-collection and processing of a biofluid. In particular, the biofluid is a sample of fluid in which testing for the presence or absence of a biological condition is desired. The biological condition may be the presence of absence of a biological specimen, such as a virus, bacteria, fungus, pathogen or disease-causing organism. The biological specimen may be a polynucleotide sequence indicative of an elevated risk of cancer or a polynucleotide sequence indicative of a disease state, including cancer. With this, the devices are compatible with detecting the presence or absence of a biomarker reflecting the presence or absence of the biological specimen.

The device may comprise a mouthpiece and a collection tube fluidically connected to the mouthpiece. In this manner, the user can spit into the mouthpiece and the collection tube can then collect the saliva. A tube insert is positioned in the collection tube, wherein the tube insert comprises a piercing end. A cap is configured to connect to the collection tube and fluidically seal the collection tube from a surrounding environment. The cap comprises an integrated reservoir configured to hold a biological reagent configured for use with the biological specimen in the collection tube. The biological reagent is selected depending on the application of interest, including the type of biological specimen, the timing of the processing/handling of the sample, and the type of biomarker and biomarker detector(s). A reservoir membrane contains the biological reagent in the integrated reservoir and temporarily seals the integrated reservoir from the collection tube. The "temporarily seals" reflects that the device has a tube insert piercing end configured to pierce the reservoir membrane, and in this manner, fluidically contact the biofluid in the collection tube and the biological reagent in the cap reservoir. This configuration advantageously ensures that none of the fluids, and constituents thereof, are exposed to the external environment. This assists in ensuring safety of users and others around the device and assists in maintaining biofluid integrity without possible external contaminants.

The tube insert may further comprise a positioning feature to align and position the piercing end at a pre-determined distance from the reservoir membrane and to preserve a detection interface with a detection membrane.

The device may further comprise a detection membrane positioned at the bottom of the tube insert; a means for driving fluid flow through the detection membrane to obtain a biological measurement; and a holder for holding the detection membrane. The means may be as straightforward as being driven by gravity, flow driven by shaking of the device by a user, or an actively provided force, such as via a pressure-generated flow exerted by, for example, a syringe plunger motion or by a pump.

The reservoir membrane may be a re-sealable membrane.

The device, useful for testing, comprises a detection membrane, wherein the reservoir membrane is configured to be removable after the biological reagent is introduced to the biofluid in the collection tube; and wherein the detection membrane is a biomarker detection membrane configured to connect to the cap after the reservoir membrane is removed to detect one or more biomarkers in the biofluid.

The re-sealable membrane may comprise an interchangeable adhesive layer; and a porous substrate comprising biomarker detectors. For example, the re-sealable membrane may itself be replaceable, so that the rest of the device could be reused. This could be particularly relevant if the same user would like to use the device for multiple serial tests, such as a repeat for a different biomarker. By simply swapping out the detection membrane, another sample can be provided for immediate testing. Accordingly, "re-sealable" is used broadly herein to refer to a membrane that can be removed and replaced without sacrificing device accuracy or reliability. This reflects that the reservoir membrane itself could correspond to the detection membrane. Of course, the membranes can also be distinct membranes, including physically separated or in a stacked configuration.

The biomarker detectors, depending on the application of interest, may be selected from the group consisting of polynucleotides, polypeptides, antibodies, nucleic acids, toxins, bacteria, virus, and biological vesicles.

The devices provided herein are compatible with multiplex detection of a plurality of biomarkers. For example, the device may comprise a plurality of unique biomarker detectors arranged in a microarray on the detection membrane for multiplex detection of biomarkers in a biofluid.

The device may further comprise a vertical flow immunoassay (VFI) and/or a lateral flow assay (LFA) integrated with the reservoir membrane or a detection membrane, including a detection membrane that is a VFI or LFA membrane comprising one or more biomarker detection elements, optionally the biomarker detection elements are antibodies.

The vertical flow immunoassay may comprise a sandwich enzyme-linked immunosorbent assay (ELISA). In a VFI assay, provided is a means for driving fluid flow through the vertical flow substrate membrane; wherein the device is configured to reflect presence of the biomarker by a change in optical color at the membrane. The change in color may be by binding of optical markers that change color of the membrane upon binding. Again, the means for driving fluid flow may be by any of a passive means (inverting to ensure flow under gravity through the membrane), active (by user-supplied force or by an electronically driven pressure controller to drive fluid flow across the membrane).

The tube insert may comprise an insert body shaped for insertion into a reservoir of the collection tube; a flange on the insert body configured to be supported by an entrance wall of the collection tube; a bottom surface having a central opening; wherein the piercing end corresponds to a spike extending from the bottom surface. This combination of enhanced structure to the tube insert is beneficial for more precise puncture of the reservoir membrane and device robustness.

The piercing end may correspond to a plurality of physically separated spikes. In this manner, piercing reliability is increased and flow exchange maximized.

Also provided herein are methods of using any of the described devices for self-collection and processing of a biological specimen, including a biological specimen that may be found in a biofluid.

For example, the method for self-collecting and processing a biofluid, including a biofluid that may have a biological specimen, may comprise the steps of providing the device and introducing the biofluid into the collection tube by the mouthpiece. The cap is connected to the collection tube, thereby piercing the reservoir membrane with the tube insert piercing end. The biofluid is mixed with the biological reagent to obtain a mixture of biofluid and biological reagent. In this manner, the biofluid is collected, and any biological specimen in the biofluid is processed.

The method may further comprise the step of detecting for the presence of a biological interaction between the detection membrane and a biomarker of a biological specimen. In this aspect, the device is an assay for determining, for example, whether the user has a biological specimen. For example, a pathogen such as a virus that can be found in the saliva, or at least a biomarker of the virus can be found in saliva.

The method may further comprise the step of determining a presence or an absence of the biological specimen from the user who provided the biofluid by introducing the biofluid to the detection membrane.

The method may further comprise the step of determining a physiological parameter from the biofluid for self-diagnostics of a health condition, including by introducing the biofluid to the detection membrane.

The tube insert may be positioned in the collection tube before the step of introducing the biofluid into the collection tube.

The method may further comprise the steps of removing the cap from the collection tube, exchanging the reservoir membrane with a biomarker detection membrane, and connecting the cap with the biomarker detection membrane to the collection tube. The collection tube may be inverted to flow the mixed biofluid and biological reagent through the biomarker detection membrane under gravity or fluid impulse by shaking. The presence or absence of the biomarker in the biofluid may be detected by detecting a physio-chemical property perturbation such as by a color change and/or an electrochemical change by an electrochemical reaction that activates or releases a volatile compound in or from the biomarker detection membrane.

The biomarker detection membrane may comprise an array of detection spots, including optionally an array for multiplex detection of a plurality of unique biomarkers.

To ensure device fidelity and functionality, the biomarker detection membrane may comprise a positive control that reflects biofluid was successfully provide to the detection membrane. If the positive control does not, for example, cause a localized color change, the assay can be identified as not correctly functioning.

The method may further comprise detecting the color change by an imaging device, including a digital imaging capture device, including a camera from a smart phone, and analyzing the detected color change from the imaging device with a data analysis algorithm software to quantify a color change parameter, including intensity, color wavelength, and distribution thereof.

Also provided herein are devices for carrying out any of the methods described herein.

Also provided herein is a device for mixing two separate fluid components. The device may comprise a collection tube for containing a fluid sample and a tube insert positioned in the collection tube, wherein the tube insert may comprise: a piercing end. A cap is configured to connect to the collection tube and fluidically seal the collection tube from a surrounding environment. The cap may comprise an integrated reservoir configured to hold a sample reagent configured for use with the fluid sample in the collection tube. A reservoir membrane seals the reservoir from the collection tube. In this manner, the tube insert piercing end is configured to pierce the reservoir membrane and fluidically contact the fluid sample in the collection tube with the sample reagent in the cap reservoir.

Any of the devices provided herein may use a fluid sample that comprises saliva with a sample reagent that comprises a buffer for chemically stabilizing and/or processing the saliva.

Any of the methods and devices may integrate a detection test with the cap. For example, specially configured membranes and/or buffers may be used to facilitate sample processing and to obtain a sample measurement, including for example, presence or absence of a biomarker, such as a protein, antibody, polynucleotide, or fragments thereof. An exemplary buffer is provided in **Table** 24. Any of the methods and devices may contain such a buffer in the cap integrated reservoir. Of course, the devices and methods are compatible with a range of buffers, wherein the buffers are specifically tailored for the application of interest.

### BRIEF DESCRIPTION OF THE SEVERAL VIEWS OF THE DRAWING(S)

**FIG. 1** is a photograph of the various components of a self-collection device, including a mouthpiece and a cap containing fluid in a cap reservoir sealed with a membrane.
**FIG. 2** is a close-up photograph of the various components of **FIG. 1****,** including a collection tube with cap, collection tube with insertable tube, and the cap with the membrane and a punctured membrane.
**FIG. 3** are photographs of a threaded cap design with a reservoir.
**FIG. 4** illustrates a cap with a biomarker detecting membrane having capture antibodies configured to generate a detectable antigen-antibody complex.
**FIG. 5** is a schematic of a device for self-collection of a biofluid, including saliva.
**FIG. 6** is a schematic of a cap and tube insert, including various cross-sections to illustrate the piercing end of the tube insert that can pierce a membrane to access fluid in the cap integrated reservoir. In this example, the cap is configured to hold up to 5 mL solution.
**FIG. 7** is a schematic of a cap with threads to grip and connect to a collection tube. An enlarged flange improves a membrane seal, thereby reliably containing a biological reagent in the cap integrated reservoir.
**FIG. 8** is a schematic of a device for self-collection of a biofluid comprising a cap and collection tube with tube insert configured to pierce a reservoir membrane of the cap to fluidically access the cap integrated reservoir. The piercing end of the tube insert has a plurality of spikes. The schematic is for a 15 mL collection tube.
**FIG. 9** is a schematic of a tube insert with a piercing end corresponding to three prongs or spikes extending a longitudinal distance for piercing a cap membrane after a cap is connected to a collection tube. The tube insert has an access opening to ensure fluid mixing between the fluid sample in the collection tube and the sample reagent in the cap integrated reservoir.
**FIG. 10** is a schematic of a tube insert having a single spike piercing end to facilitate higher flow between fluids stored in the cap integrated reservoir and the collection tube reservoir.
**FIG. 11** is a schematic of a tube insert having a single prong or spike to pierce the membrane. The illustrated configuration, however, did not reliably puncture the membrane upon cap connection to the collection tube.
**FIG. 12** is a schematic of a tube insert with three prong or spikes having a more angled straight-blade configuration, for improved piercing characteristics. To prevent cap from removing the tube insert, to tope edge of the tube insert that rests on the collection tube opening edge is retracted from the edge by about 0.5 mm.
**FIG. 13** is a schematic of a mouthpiece with vents.
**FIG. 14** is a schematic of a mouthpiece without vents and with a small bead (knob) repeated in two points around the inside of the tube seat to create a three-point "pinch" for reliable snap-fit to the collection tube for use by an individual self-collecting a biofluid, such as saliva.
**FIG. 15** illustrates a vertical flow immunoassay (VFI) membrane with an array of spots and a capillary pump that is one means for driving fluid flow. Celova^{®} microfibrillated cellulose (Weidmann Holding AG, Rappeswill, Switzerland) is an exemplary pad material.
**FIG. 16** illustrates a VFI cap test dual tube configuration.
**FIG. 17** is a VFI cap-on-cap design.
**FIG. 18** illustrates a VFI liquid pump.
**FIG. 19** illustrates a VFI passive fluid pump, based on an artificial tree principle. See, e.g., Shi, W., Dalrymple, R.M., McKenny, C.J. et al. Passive water ascent in a tall, scalable synthetic tree. Sci Rep 10, 230 (2020). https://doi.org/10.1038/s41598-019-57109-z.
**FIG. 20** is a representative illustration of an integrated VFI device.
**FIG 21**is an overview of a saliva kit.
**FIG. 22** illustrates interaction of a tube insert, with a close-up view of a flange, inner surface, seal and tube insert. The flange may be 0.6 mm, with adjustment to the material/thickness to help facilitate press fit rigidity. Inner surface may have a minimal taper, such as bout 0.5° to 1°, to help maintain a more nominal wall, and benefit the eventual seal to the reagent closure. A lead-in at the location corresponding to the labeled "tube insert" can assist in assembly and reduce risk of damage and/or unwanted leak path.
**FIG. 23** illustrates a mouthpiece funnel to tube (and insert) connection. Snap beads are positioned into an upper recess of the mouth funnel. Alternatively, the fit may be a tight clearance fit, with lead-ins on the bottom of the mouth funnel, with added small crush ribs vertically within the inner column (in direction of pull) in order to facilitate a snug fit.
**FIG. 24** illustrates a reagent closure to 5 mL tube (and insert) connection. To ensure a user-applied torque creates a sufficient and reliable seal, a plug seal approach may be used, with a shallower angle of attack (less than 5°).
**FIG. 25** illustrates considerations for piercing of the reservoir membrane to the reagent in the integrated reservoir. Preferably, at least one full thread is engaged before piercing.
**FIG. 26** illustrates some embodiments to minimize excessive flex and breakage, including by connecting a plurality of piercing elements, such as by a ring connector the connect a piercing member of the tube insert with at least one or more adjacent piercing members. In this manner, uncontrolled and unwanted movement of the piercing end is avoided, thereby providing more reliable membrane piercing.
**FIG. 27** illustrates a representative device.
**FIG. 28** illustrates a VFI membrane and pad materials for a biomarker detection assay, including antibodies and/or genes.
**FIG. 29** illustrates a cap of a representative device.
**FIG. 30** illustrates a cap of a representative device.
**FIG. 31** is a schematic of a cap of a representative device.
**FIG. 32** illustrates a cap of a representative device.
**FIG. 33** illustrates a collection tube of a representative device.
**FIG. 34** illustrates a representative device.
**FIG. 35** illustrates a representative device.
**FIG. 36** is an image of usability questionnaire related to a representative device.

### DETAILED DESCRIPTION OF THE INVENTION

The following examples further illustrate the invention but, of course, should not be construed as in any way limiting its scope.

"Self-collection" refers to the ability of a user, even a non-medically trained user, to reliably and safely provide a biofluid in a manner conducive to subsequent testing. No other persons are required to obtain the biofluid that is tested, either immediately, or at a later time. In this manner, the devices provided herein can be extensively distributed, including to homes, offices and worksites, where the users at those locations can self-collect a biofluid for testing. For example, self-collection of saliva to determine the presence or absence of a pathogen that can be found in saliva. Of current relevance are viruses that cause coronavirus disease (e.g., SARS-CoV-2 virus that causes COVID-19). Of course, the devices provided herein are compatible with any pathogens for which biomarker detectors are available. This typically involves polynucleotide sequences that are associated with a specific pathogen, such that complementary biomarker detectors (e.g., complementary polynucleotide sequences; capture antibodies) can be incorporated with a detection membrane.

The term "processing" is used broadly herein and refers to an action on the fluid sample useful in an assay. For example, processing can correspond to one or more of lysis, nucleic acid extraction or stabilization, as well as analysis, including in embodiments having an antibody array(s) for detecting protein or viral particles in a sample, including in a COVID test. For devices that are configured to test for COVID, the biomarker detectors may correspond to any antibodies that specifically detect a protein associated with a virus responsible for COVID, including, but not limited to, the list of antibodies against SARS-CoV-2 S protein provided in **Table 25.**

"Fluidically connected" refers to the connection of two components so that fluid flow may occur between them, but without adversely impacting the functionality of each component.

### EXAMPLE 1

This example demonstrates an assay incorporated with the cap to provide a biofluid collection device with an optional assay for detecting presence or absence of a biological specimen or a biomarker in the biofluid.

In this example, a re-sealable membrane can also comprise an interchangeable adhesive layer holding a porous material, for example paper filter with pore sizes of at least 10 nm, preferably in the range of about 100 nm to a few microns, which can comprise an array of spots for testing biomarkers. The tests could comprise molecular detection of proteins, antibodies, nucleic acids (e.g. RNA or DNA) to detect viruses, bacteria but also enzymes, circulating microvesicles (e.g. exosomes) or other chemical products of a biological reactions.

For example, the membrane can be printed with antibodies for detecting a sandwich assay, ELISA like or other colorimetric test for investigating biofluids, e.g. saliva, blood, urine, ascites or other biological sample for assessing the presence of target analytes, e.g. virus, bacteria, toxins.

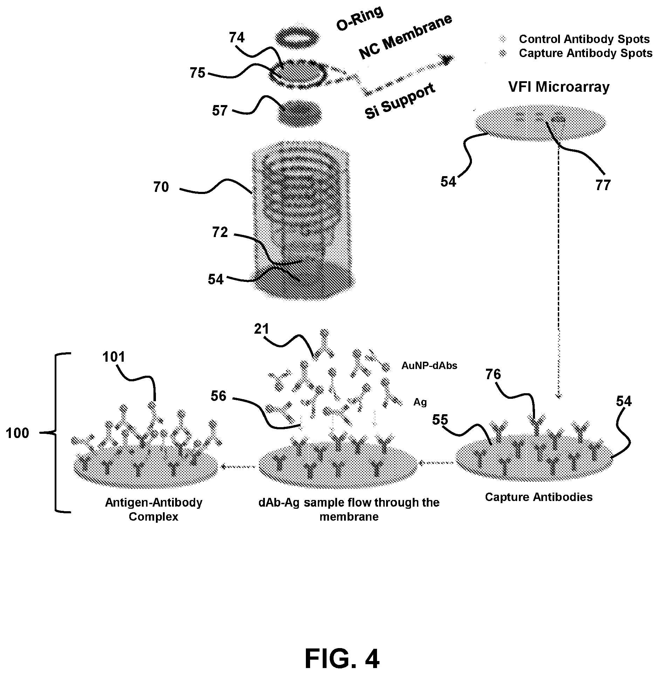

As illustrated in **FIG. 4****,** a vertical flow immunoassays **100** can be integrated onto the membrane of the cap **70,** as illustrated by detection membrane **54.** Detection membrane has a detection interface **55** corresponding to the surface to which biomarker detectors **76** are connected, including laid out in a microarray **77** of biomarker detectors. **FIG. 4****,** for convenience, illustrates biomarkers as capture antibodies. Of course, the systems provided herein are compatible with any of a range of biomarker detectors, including polynucleotide, polypeptides and the like, so long as there is specificity for a biomarker **21** (e.g., reflective of a biological specimen of interest) of interest that is being tested for in the biofluid. The detection membrane **54** may be held in place by a holder **57,** illustrated as a Si-Support and further support provided by a porous substrate **75,** illustrated as a nitrocellulose (NC) membrane. To facilitate fluid sealing, O-ring(s) may be used to fluidically seal the membrane in the cap with the holder **57,** such as at the bottom of the tube insert. For further reliable membrane positioning, an interchangeable adhesive layer **74** may be positioned between the holder and the detection membrane, including on the top and/or bottom surface of the porous substrate **75.** The bottom panels provide a summary of the VFI **100** structure, including biomarker **21** and means **56** for driving fluid flow through the detection membrane **54** (reflected by arrows), which may range from passive means, such as by diffusion of biomarkers and/or to more active means such as by exertion of a pressure on the top side of the membrane **54** (and/or reduction of pressure on the bottom side of membrane **54).** This may be by means of structure corresponding to an electrically powered pump in fluid contact on either side of the membrane or by a manually exerted force, such as on a syringe connected to either side of the membrane, controlled by a person. The bottom panels of **FIG. 4** illustrate a VFI that uses a sandwich ELISA **101** process for detecting a biomarker **21.** Of course, the devices provided herein are compatible with a lateral flow assay (LFA) configuration, such as by replacing the VFI membrane with a LFA membrane (capture spots arranged laterally in a fluid flow direction), where mixture is flowed in a lateral direction over the detection interface **55** (in contrast to flow direction **56).**

Typically, the assay can rely on the immobilization of a capture antibody on the sealable membrane that acts as a reagent pad to which the sample of interest (with or without antigen (e.g., biomarker) to be detected) is applied. Detection of the bound antigen is subsequently achieved through the binding of an antigen specific antibody gold conjugate. This step completes a sandwich comprising a capture antibody, an antigen and finally the gold conjugate, and results in a direct and permanent visually detectable color change, including a color dot indicating the presence of the antigen. A combination of different nanoparticle conjugate probe or nanoparticle shape and materials can be used for tuning the detection in the configuration of a multiplex assay.

In an embodiment, the detection of an antibody in a sample can be done in a few steps:

After the collection of the sample in the tube using the sample collection cup, attach the cap which is prefilled with a solution of reagents **72** containing gold conjugate.

During the attachment of the cap to the tube, the sealed membrane is pierced releasing the reagents into the tube with the sample which can be mixed by shaking the tube assembly a few times.

Remove the cap and place the adhesive membrane, which has been printed with an antigen, onto the cap.

Invert the tube and cap assembly so the sample is applied to the membrane.

Observe the change in color onto the spots of the membrane on the cap indicating the outcome of the assay.

### EXAMPLE 2

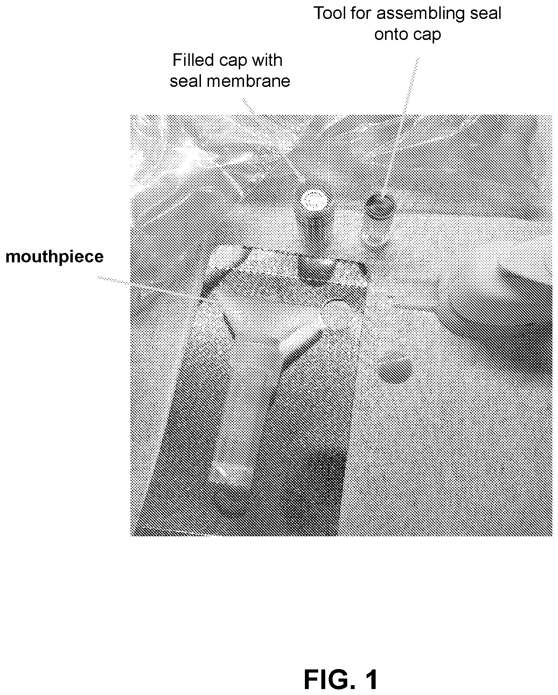

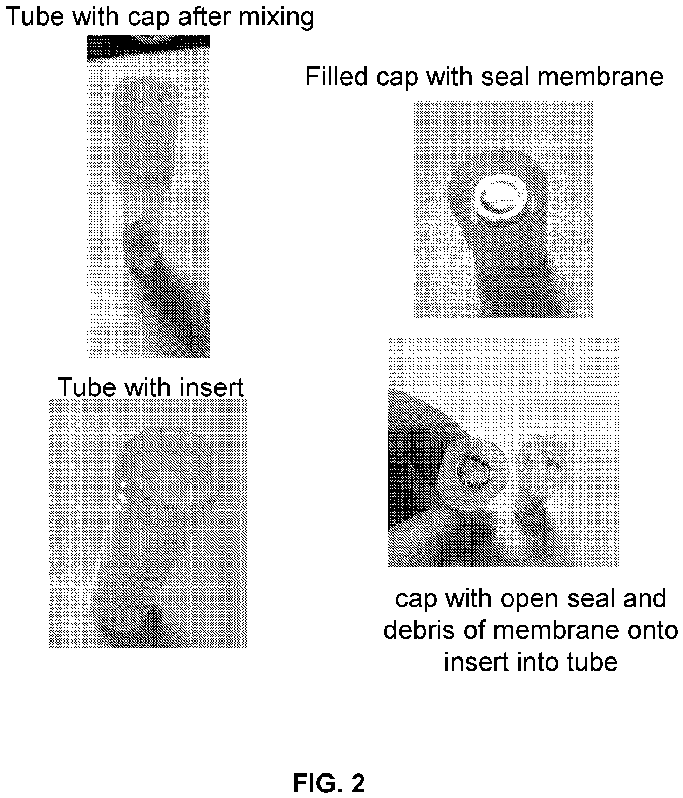

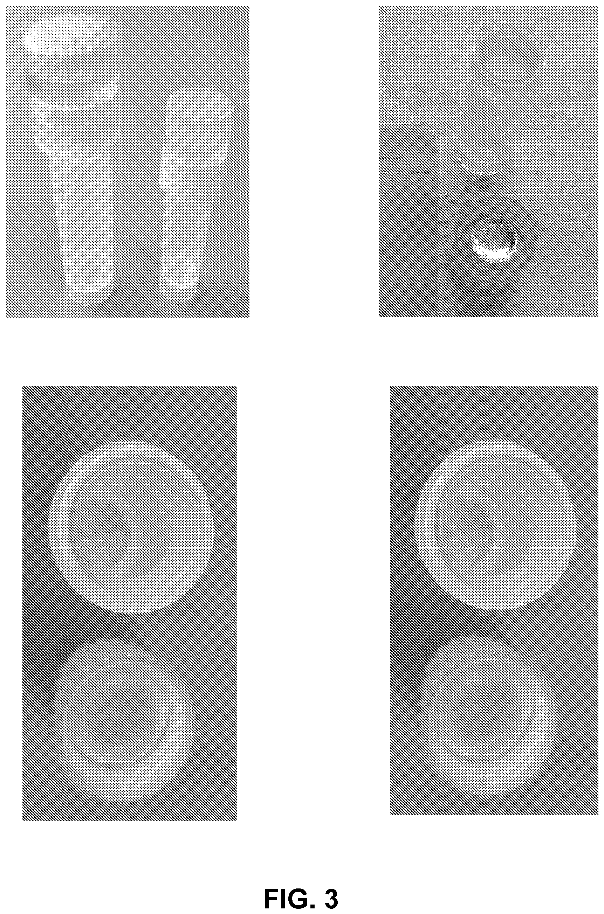

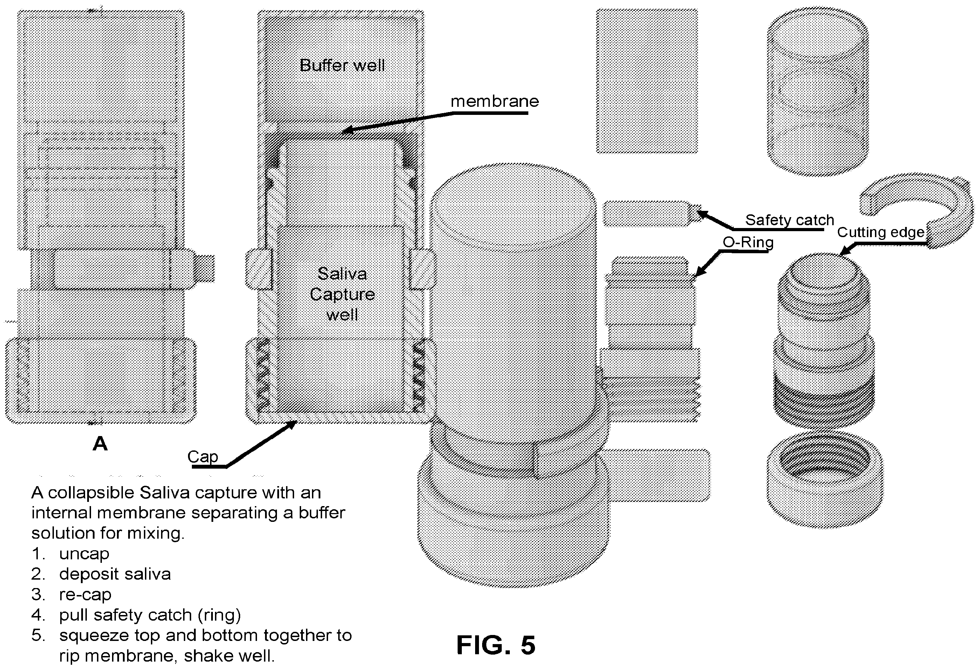

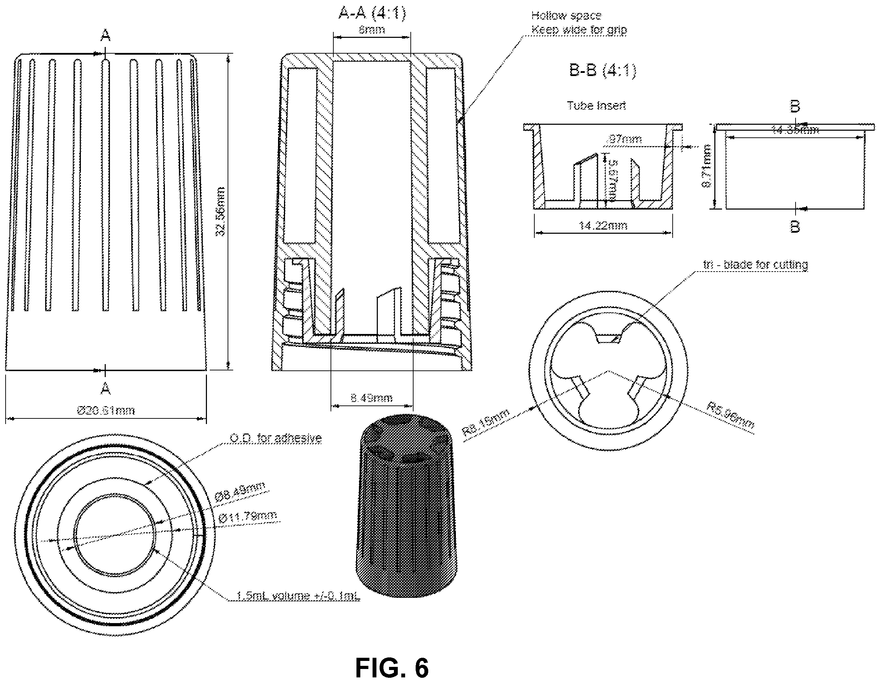

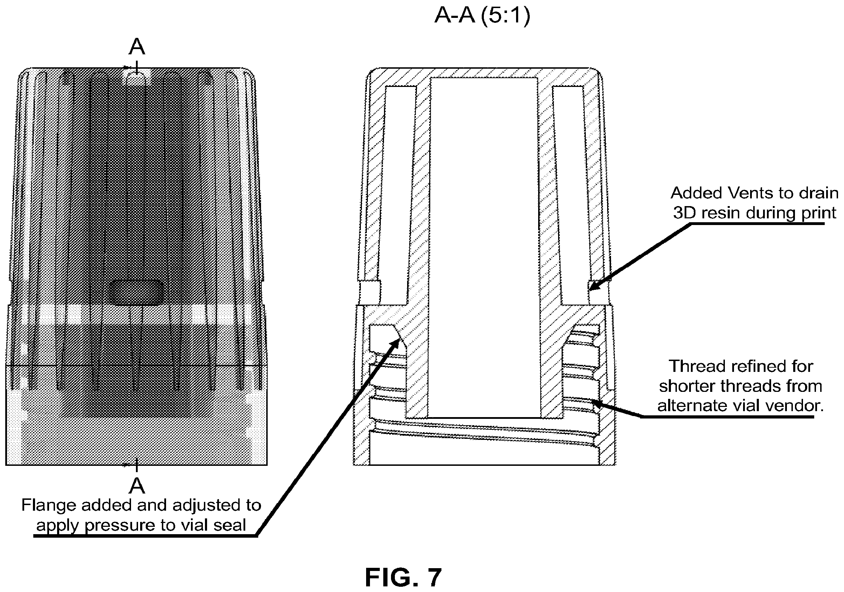

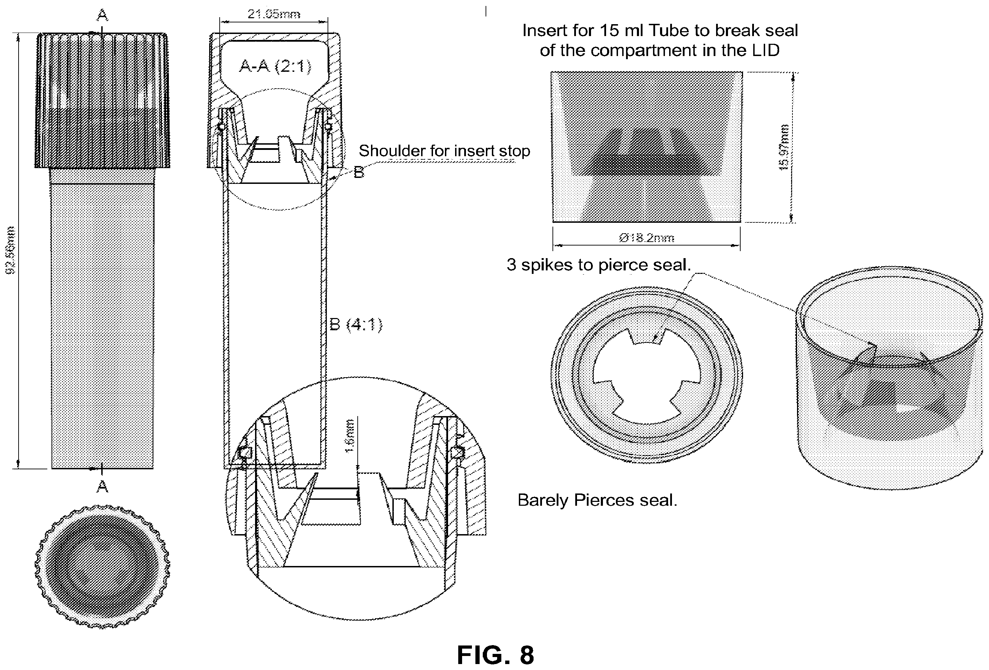

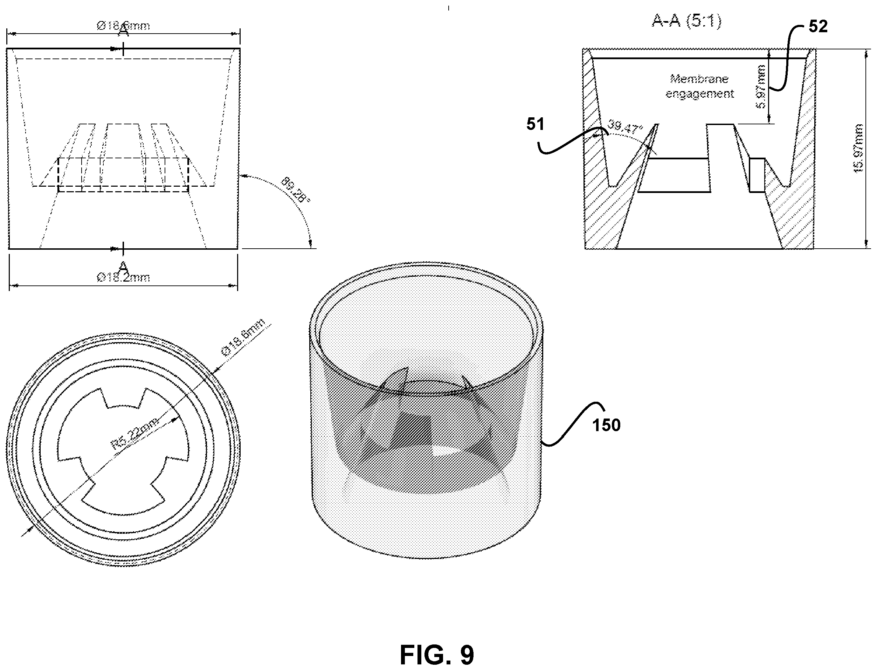

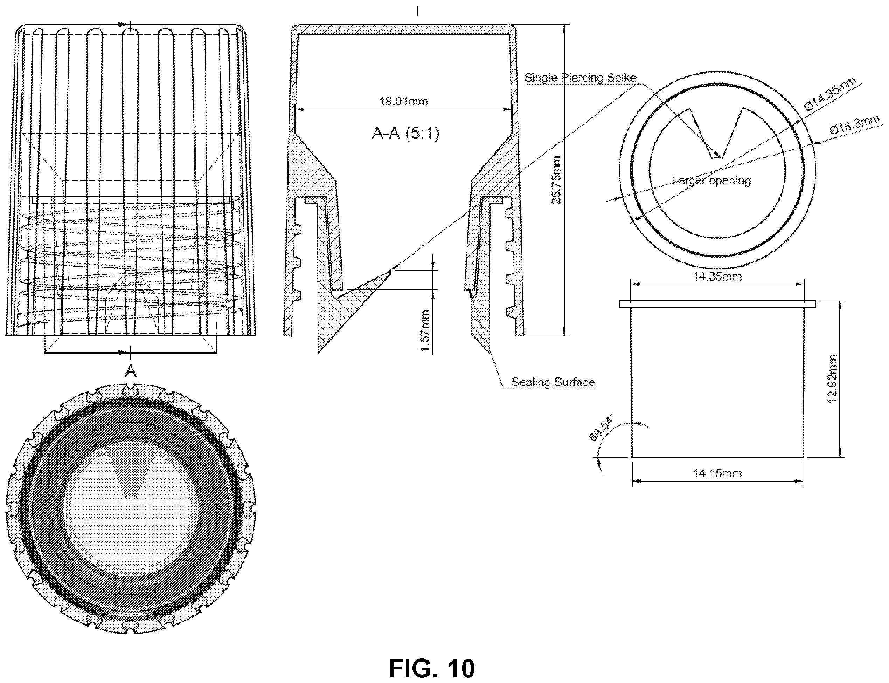

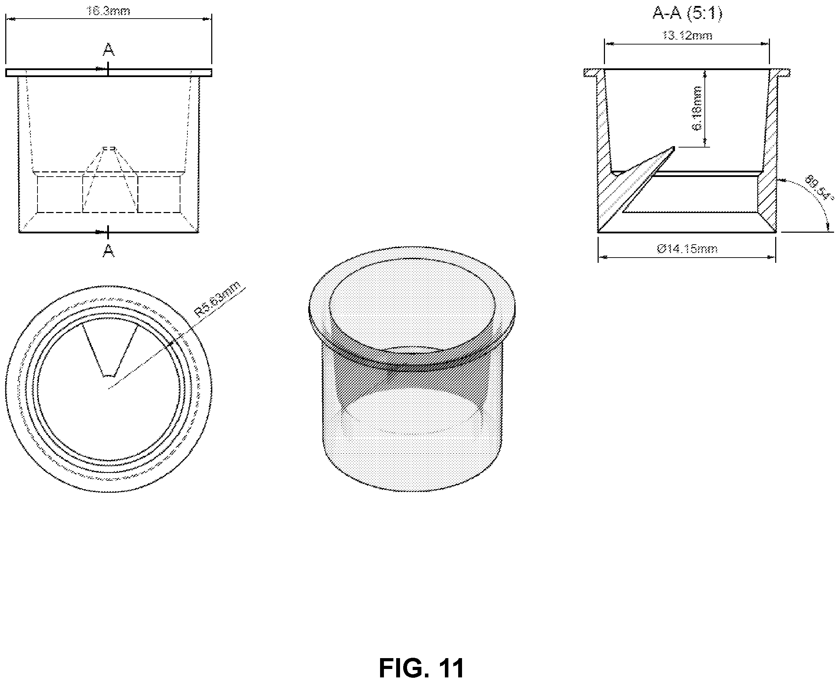

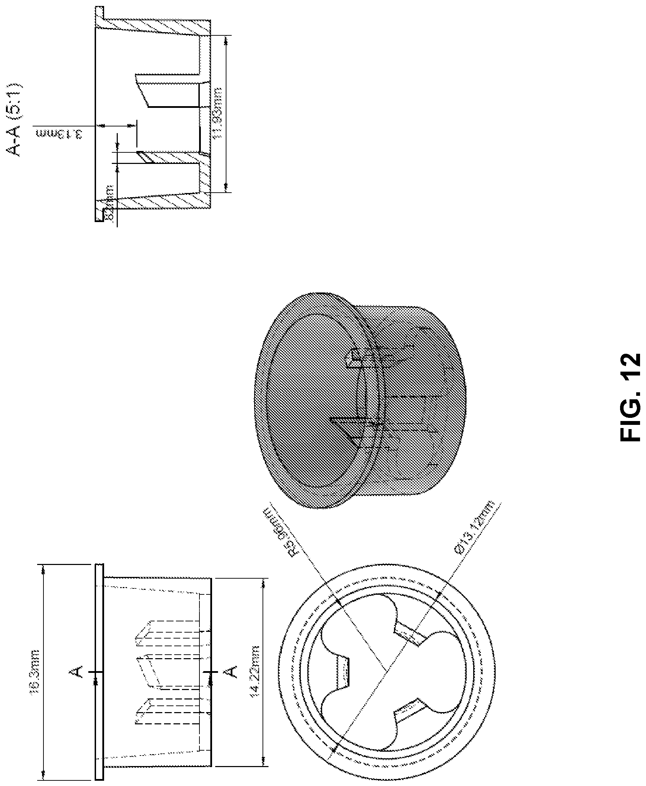

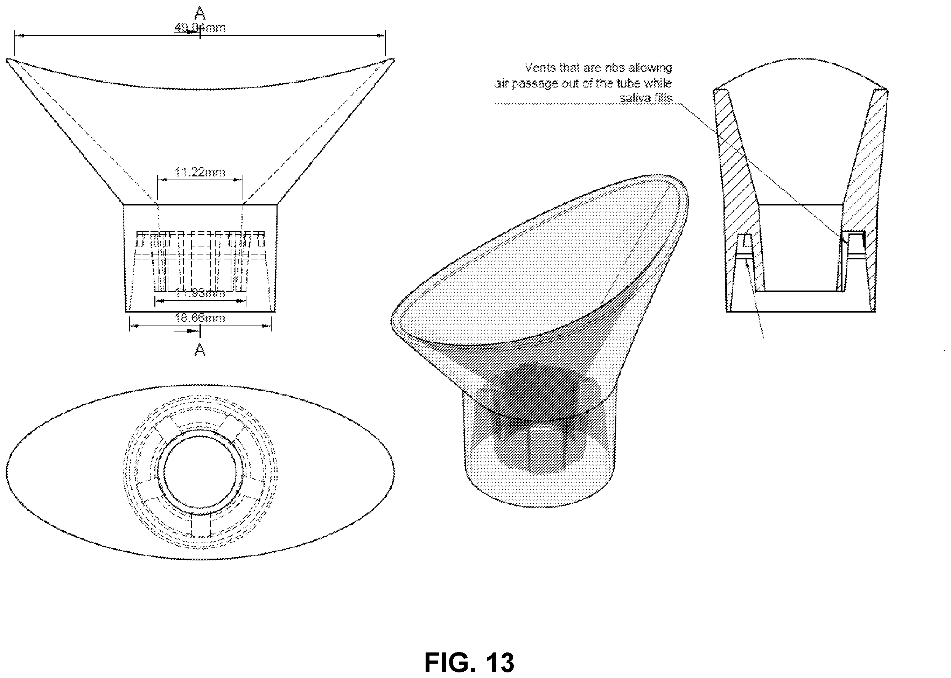

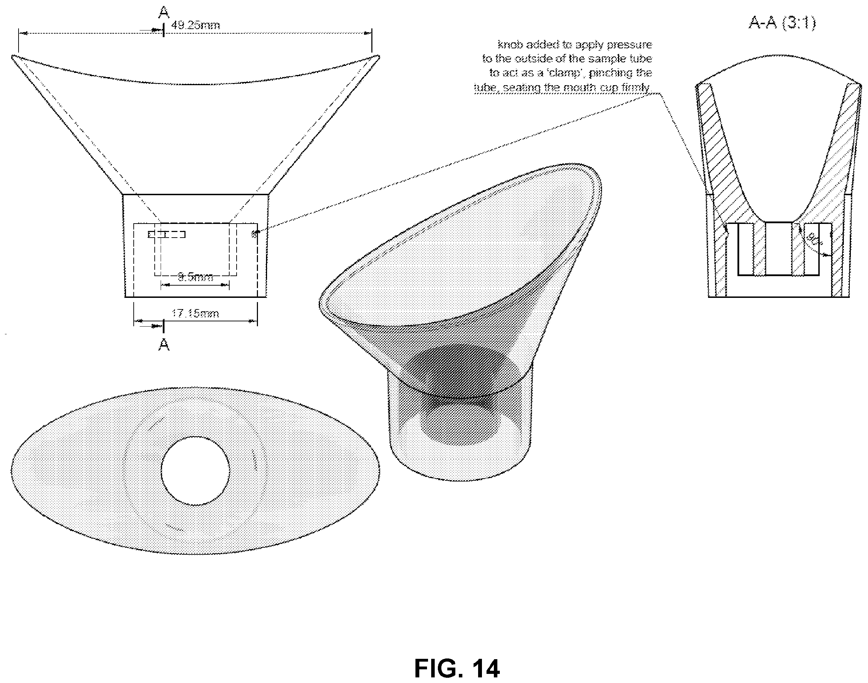

**FIGs 1-3** are photographs of exemplary devices. **FIGs 5-14** schematically illustrate the various components of a device for mixing two separate fluid components (e.g., **FIG. 8****),** and an optional mouthpiece **(****FIGs 13-14****)** that can be connected to the collection tube to make a device for self-collection of a biofluid (e.g., saliva). Exemplary illustrated components include cap **70,** collection tube **40,** tube insert **50** and mouthpiece **30,** that together provide safe and reliable self-collection of a biofluid **20,** such as saliva, along with an "automated" mixing of biological reagent contained in the cap after the cap is secured to the collection tube with tube insert. For example, the cap may be in threaded connection to the collection tube, such that as the cap is screwed onto the collection tube, the tube insert, positioned at the top portion of the collection tube, having piercing end **60,** pierces the membrane so that the biological reagent mixes with the biofluid. The tube insert **50** piercing end **60** includes positioning feature(s) **51,** illustrated as spike angle, so that there is a pre-determined distance **52** from the reservoir membrane **(****FIG. 9****).** As described, other features include the threaded connection and flange, so that the distance **52** is accurately maintained. Of course, other positioning feature structures are compatible with the device, including a press-fit connection, as well as shaping insert body **150** to ensure the insert is appropriately positioned relative to the collection tube **40.** This advantageously avoids risk of inadvertent exposure by the user to biological reagent, while also ensuring reliable and prompt mixing of reagent and sample in one single integrated device.

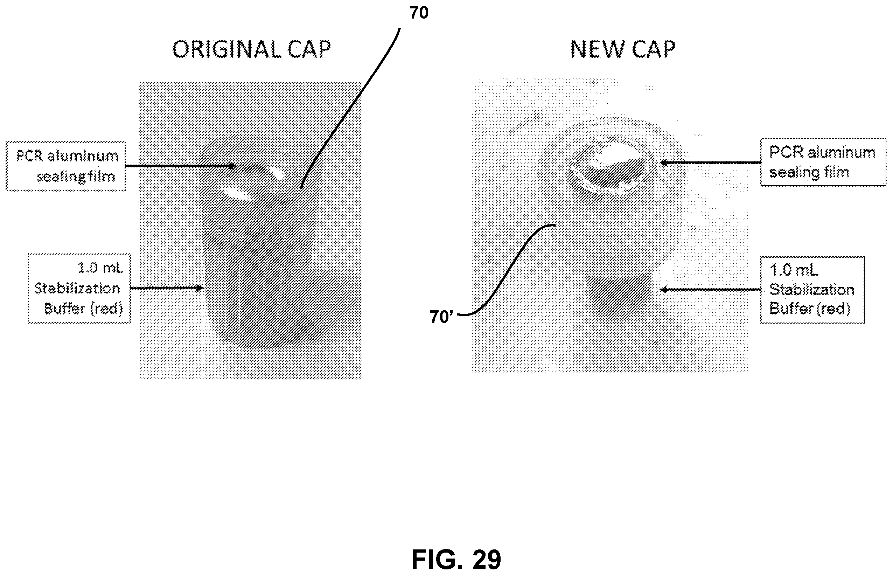

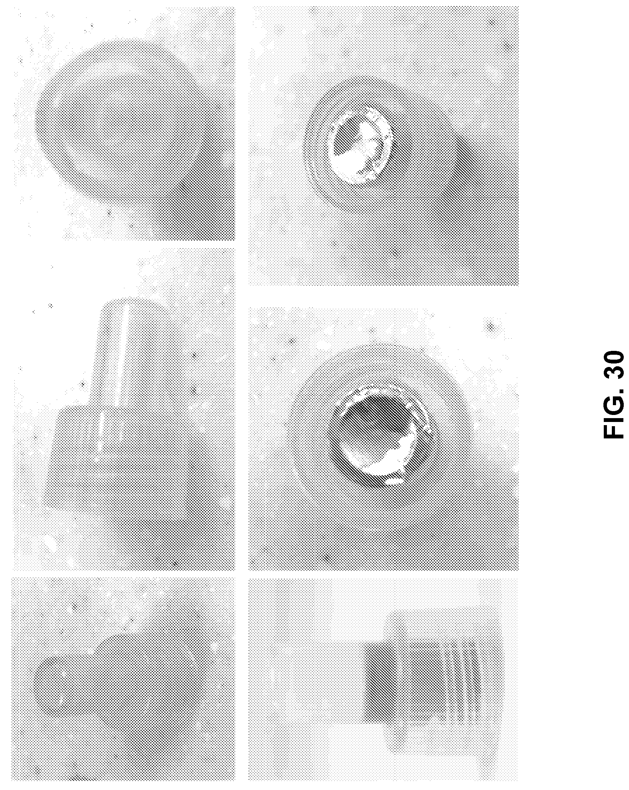

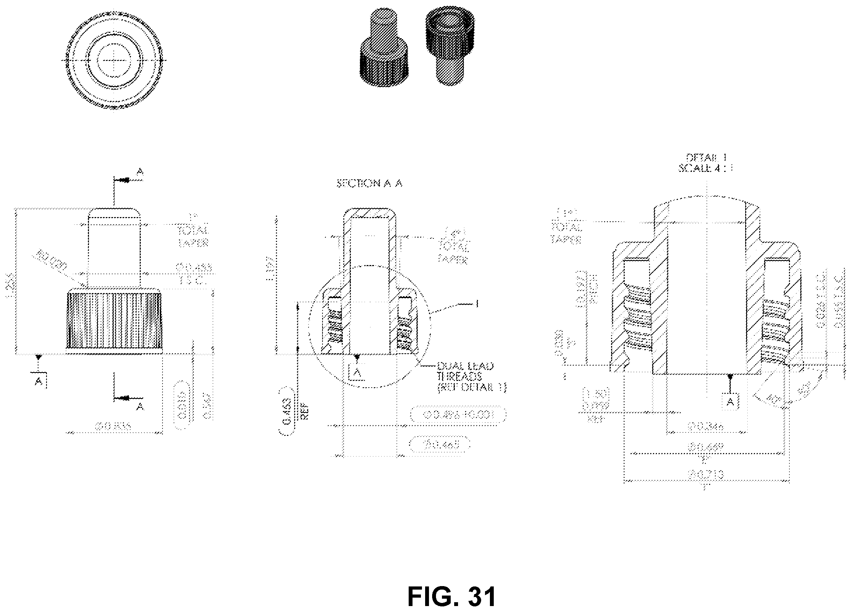

In an embodiment, the cap **70** may be configured to facilitate usability (e.g., easier to hold) and manufacturing (e.g., reduce amount of plastics). Manufacturing, in particular, is an important consideration in view of challenges in obtaining supplies, including the ongoing pandemic-related supply chain bottlenecks. As shown in **FIG. 29****,** the cap **70** of the above-described figures is presented alongside a cap **70'** designed according to the aforementioned design constraints (i.e., usability, manufacturing). Each cap enables holding of a volume of stabilization buffer, or biological reagent, and sealing of the biological reagent within a reservoir of the cap by a membrane (e.g., PCR aluminum sealing film). Cap **70'** provides a reduction in materials by ~41%. **FIG. 30** provides additional views of the cap **70',** both empty and filled with a volume of biological reagent. Engineering drawings of the cap **70'** are shown in **FIG. 31****.**

In an embodiment, the cap **70'** of **FIGs 29-31** is submitted for performance studies to assess its transport viability. Firstly, to assess watertight sealing status, vacuum testing is performed on the cap according to D6653/D6653M ASTM protocols. Subsequently, the combined cap/collection tube system are examined for functionality. Studies are undertaken at an altitude of 1,086 ft (331 m) (~99.1 kPa at 70°F [21°C]) using a vacuum system allowing testing up to 95 kPa. Pressure and timed evaluations are undertaken. As summarized in **Table 1,** various conditions are evaluated.

The results of the experiments are included in **Table 2.**

All of the caps withstood pressures up to 95 kPa for up to 24 hours. All cap/tube combinations withstood pressures up to 95 kPa for 48 hours. A failure was identified at 95 kPa at 48 hours in ~30% of the caps. Further investigation of the cap failure identified that this was due to bond failure and not the membrane itself, as the membrane does not rupture. Instead, it appears the adhesive between the membrane and the cap failed. Considered holistically, these results indicate the membrane on the cap and the capped tube exhibit significant pressure resistance.

To further evaluate the cap and the capped/tube collection under real-world conditions, additional experiments were carried out. Of note, transportation have the following characteristics: cargo air jet = 75 kPa (< 8 hours), ground transportation = 64.5 kPa maximum.

In Study 1 (*n=*50)*,* pre-filled biological reagent filled caps were transported by FedEx cross-country (from the manufacture site in Phoenix, AZ) to Branford, CT (Friday to Monday transit time). Samples were sent such that temperatures varied throughout transit from Phoenix, AZ, to Indianapolis, IN, to East Grandby, CT, and to Branford, CT. After 72 hours in transit, no evidence of seal leakage or any alteration of the sealing was identified (*n*=50). Thus, no leakage was noted in caps subjected to long distance road/air transportation.

In Study 2 (*n*=40x2), a separate clinical study was undertaken in New York, NY. 40 kits were sent on two separate occasions by overnight shipping. Tubes were routed from Connecticut via Memphis, TN (48 hours total transport). No leaks were noted in the sealed caps after arrival in New York, NY (*n*=80). After saliva collection, the collected samples were returned by overnight shipping from New York, NY to Branford, CT. In this study, a capped, sealed tube with saliva was sent by FedEx (i.e., standard approach). Samples were received within 24 hours. No leakage of contents was identified (*n*=80). Watertight seal (cap/saliva tube with clinical samples) was identified in 100%. Thus, no leakage of contents was identified on the first phase of transport (send-out) nor on the second (returning clinical samples).

In Study 3 (*n*=155)*,* usability studies were performed using 155 samples that were collected and returned to Branford, CT by overnight FedEx from two different sites in CT. In these instances, a capped, sealed tube with saliva (i.e., real-world assessment) was sent by FedEx. No leakage of contents was identified in any of the 155 tubes.

Taken together, these 3 studies identify that in formal assessment using real-world clinical conditions the cap seal is robust (*n*=285). They also confirm that clinical samples (capped tube with clinical material/saliva) can be transported safely without breakage or leakage (*n*=285). These data demonstrate the integrity of both the membrane on the cap and the capped tube under real-world conditions.

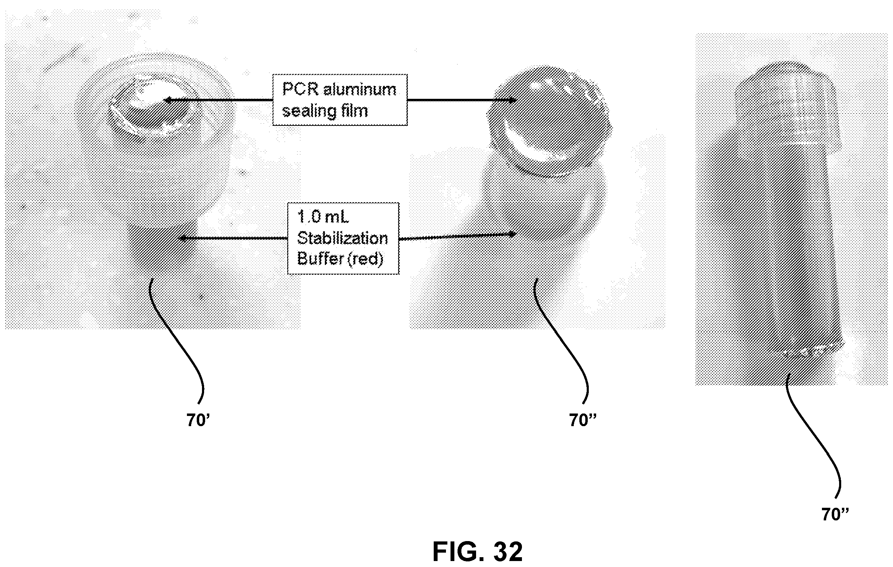

In an embodiment, the cap can be configured for robotic and high through-put usability (e.g., easier to include in robotic tube racks). As in **FIG. 32****,** a comparative image of a cap **70'** and a cap **70"** elucidate the differences therebetween. For instance, the cap **70"** incudes a biological reagent reservoir (i.e., stabilization buffer reservoir) extending toward the direction of linear motion generated when the cap **70"** is mated with the collection tube. This can be appreciated in contrast to the cap **70'** where the biological reagent reservoir extends away from the direction of linear motion generated when the cap **70'** is mated with the collection tube.

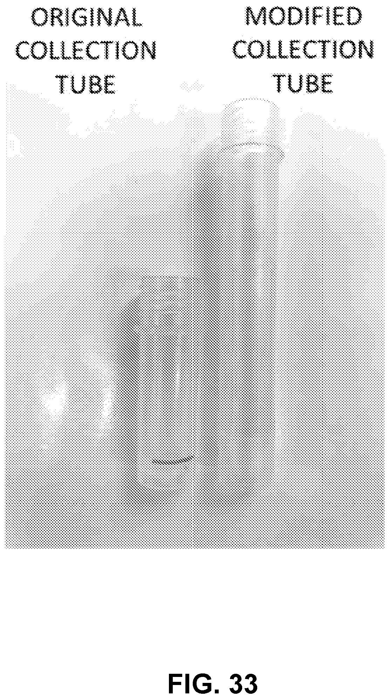

In an embodiment, the collection tube may be modified as shown in **FIG. 33****,** wherein the modified collection tube, in comparison with the original collection tube, is modified (e.g., lengthened) in order to permit automation and robotic handling.

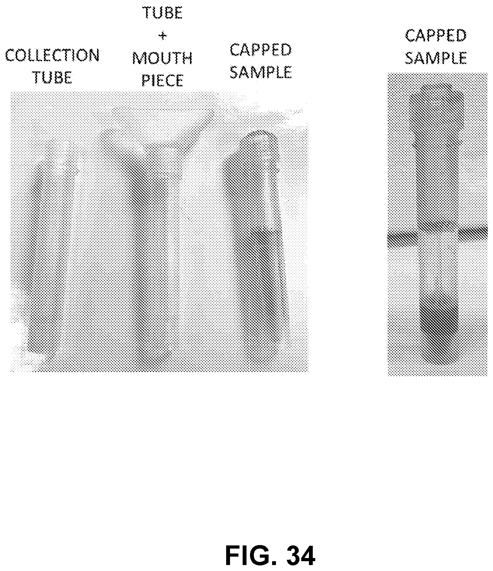

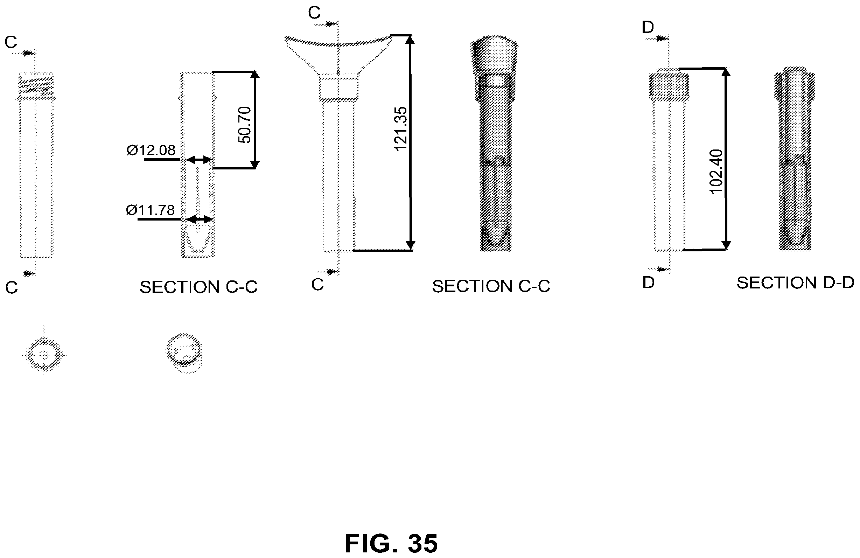

**FIG. 34** illustrates the cap **70"** of **FIG. 32** and the modified collection tube of **FIG. 33** when integrated with the methods of the present disclosure. From left, the collection tube can be first fitted with a mouthpiece to allow introduction of saliva sample. Once collected, the saliva sample can be secured within collection tube by mating the cap **70"** therewith. The far-right image shows an additional image, at a different time point, of the capped sample. Engineering drawings that accompany **FIG. 34** are shown in **FIG. 35****.**

### EXAMPLE 3 - Integrated VFI Membrane

The devices and methods provided herein are compatible with vertical flow immunoassay (VFI) membranes integrated into a saliva collection cap. Such a configuration is compatible with rapid and safe Covid-19 testing. For example, the integrated assay may detect presence or absence of antibodies, including antibodies that may be useful in a SARS-Cov-2 or COVID-19 test. The antibodies are representative examples of some useful potential biomarkers. See, e.g.:
"AI334, AQ806 and RB596 antibodies recognize the spike S protein from SARS-CoV-2 by immunofluorescence." Marchetti et al. Antibody Reports. 3: e219 (2020);
"AR222, AR249, AS274, AS702 and AS708 antibodies recognize the spike S protein from SARS-CoV-2 by immunofluorescence." Marchetti et al. Antibody Reports. 3: e228 (2020);
"AR222, AR249, AS274, AS702 and AS708 antibodies recognize the spike S protein from SARS-CoV-2 by ELISA." Hammel et al. Antibody Reports. 3: e220 (2020);
"The RB596 antibody recognizes the spike S protein from SARS-CoV-2 by ELISA." Hammel et al. Antibody Reports. 3: e218 (2020);
"RB572, RB574 and RB576 antibodies recognize the membrane M protein from SARS-CoV-2 by immunofluorescence." Marchetti et al. Antibody Reports. 3: e231 (2020);
"RB579, RB580, RB581, RB582, RB583, RB584 and RB585 antibodies recognize a peptide of the SARS-CoV-2 E protein by ELISA." Marchetti et al. Antibody Reports. 3: e191 (2020);
"RB620 and RB621 antibodies recognize a peptide of the SARS-CoV-2 E protein by ELISA." Hammel et al. Antibody Reports. 3: e238 (2020);
"The RB621 antibody recognizes the envelope E protein from SARS-CoV-2 by immunofluorescence." Marchetti et al. Antibody Reports. 3: e239 (2020);
which disclose various antibodies that can be used in any of the devices and methods described herein, including in a VFI membrane.

The cap may incorporate pad materials used in the cap. The pad materials may be characterized as having pore sizes, such as pore sizes from 10 nm -200 microns. Preliminary data suggests 80 nm may optimum.

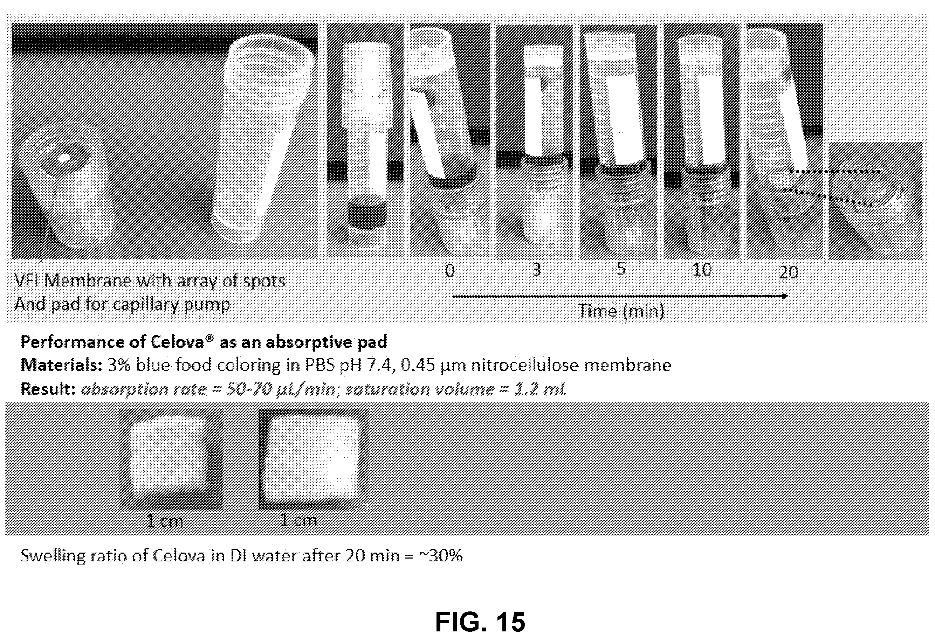

The pad materials may comprise cellulose micro-fibrils such as a Celova^{®} material. **FIG. 15****.** The pad material may be tuned, such as to have a selected pore size around 80 nm or within a sub-range of 10 nm to 200 µm, or between 10 µm to 200 µm. See, e.g., "Ultra-Porous Nanocellulose Foams: A Facile and Scalable Fabrication Approach" Antonini et al. Nanomaterials 9: 1142 (2019).

The systems provided herein are uniquely configured for the potential dual use of the biofluid processing with the buffers and the ability to run a multiplex assay within the same device. The VFI platform is compatible with gene testing, including pandemic-relevant genes, associated with influenza, Covid-19, Ebola and the like.

Ahead of efficient tests for such genes, however, is to obtain an approved saliva collection device, such as an FDA EUA device that is mass producible.

### EXAMPLE 4 - Integrated VFI Membrane

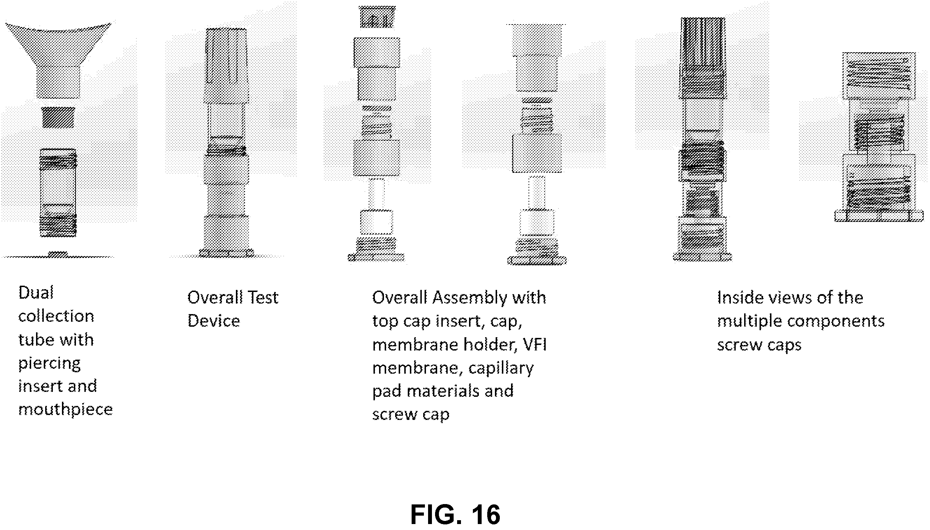

**FIGs. 16-28** illustrate various aspects of an integrated VFI membrane, a VFI cap test dual tube configuration.

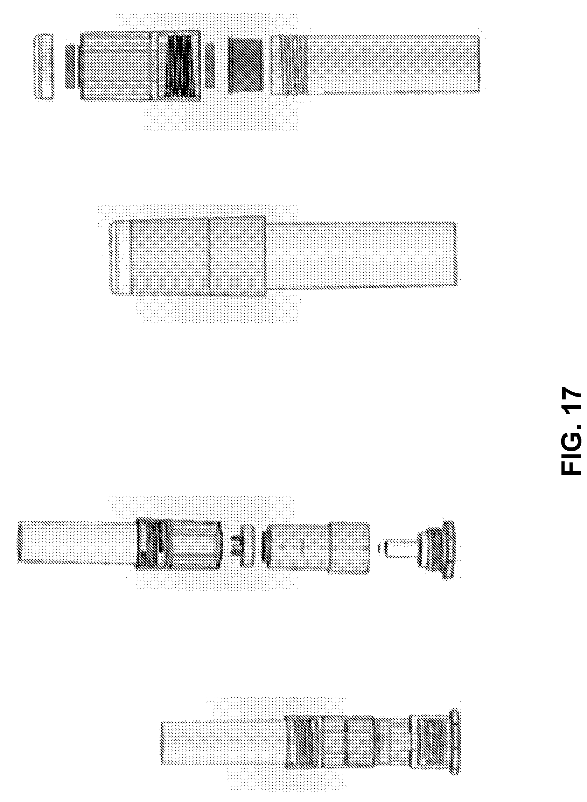

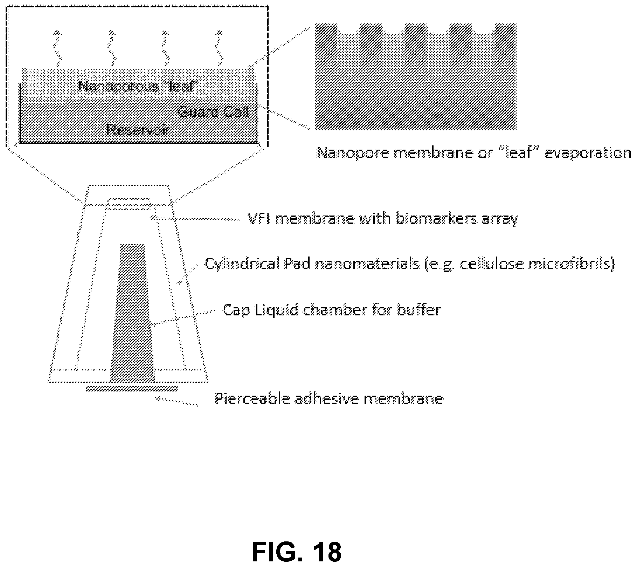

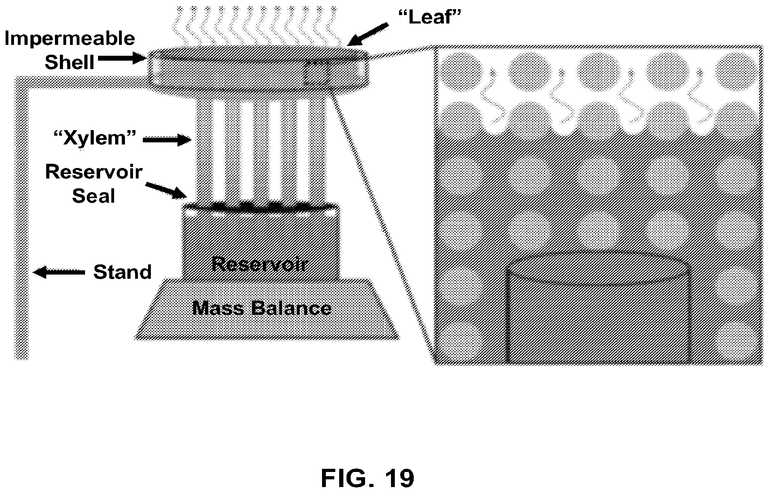

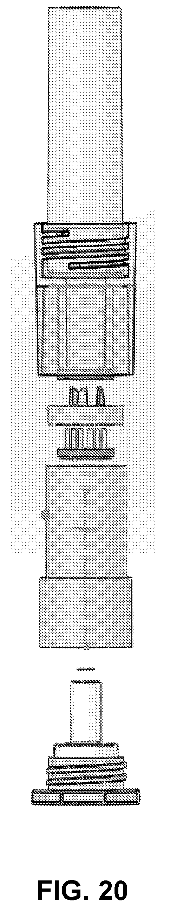

**FIG. 16** illustrates a VFI cap test dual tube configuration and **FIG. 17** is a VFI cap-on-cap design. **FIG. 18** illustrates a VFI liquid pump and **FIG. 19****,** in contrast, illustrates a VFI passive fluid pump, based on an artificial tree principle. See, e.g., Shi, W., Dalrymple, R.M., McKenny, C.J. et al. Passive water ascent in a tall, scalable synthetic tree. Sci Rep 10, 230 (2020). https://doi.org/10.1038/s41598-019-57109-z. **FIG. 20** is a representative illustration of an integrated VFI device.

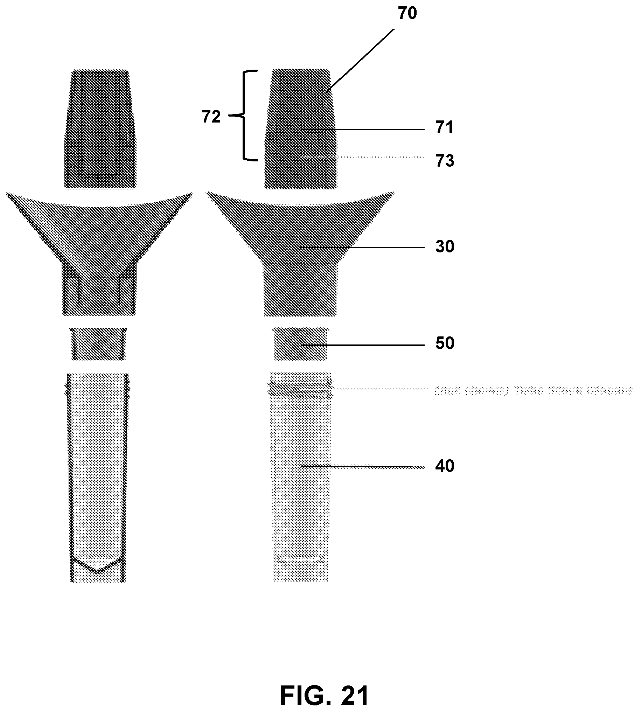

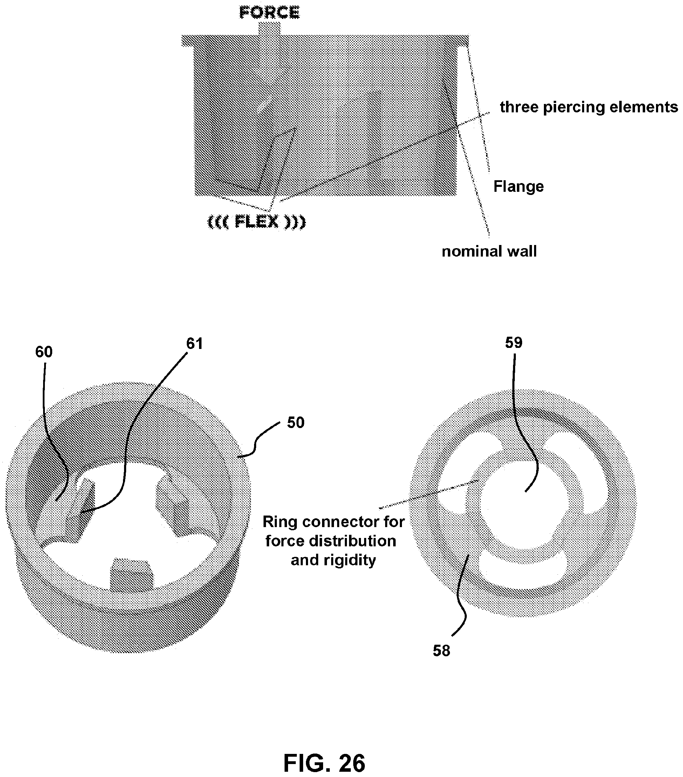

**FIG 21** an overview of a device **10** for self-collection of a biofluid, including specifically saliva. Illustrated components of device **10** include mouthpiece **30** having a funnel for convenient and reliable collection of saliva from a user. A collection tube **40** may collect the saliva from the mouthpiece **30.** Representative collection tubes include standard 5 mL tubes. Tube insert **50** is illustrated between mouthpiece **30** and tube insert **40,** and may contain a piercing end **60,** including spikes **61** (see, e.g., **FIG. 26). FIG. 26** also identifies bottom surface **58** of tune insert **50** and a central opening **59,** to facilitate a more uniform force distribution during use and increased rigidity and robustness of tube insert. Cap **70** may contain an integrated reservoir **71** containing a biological reagent **72** useful in the process for identifying an analyte/biomarker reflective of a biological specimen whose presence is being tested for in the biofluid. A reservoir membrane **73** may be used to contain the biological reagent within the cap **70** integrated reservoir **71,** at least until being pierced by the piercing end **60** of tube insert **50.**

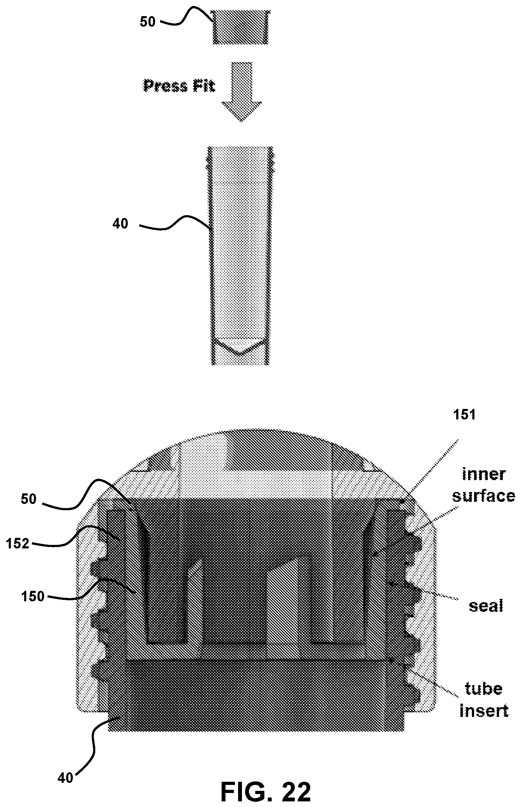

**FIG. 22** illustrates interaction of a tube insert **50** and collection tube **40** (top panel), with a close-up view (bottom panel) of a flange **151** (supported by entrance wall **152** of collection tube **40),** inner surface, seal and tube insert **50.** The flange may be 0.6 mm, with adjustment to the material/thickness to help facilitate press fit rigidity. Inner surface may have a minimal taper, such as bout 0.5° to 1°, to help maintain a more nominal wall, and benefit the eventual seal to the reagent closure. A lead-in at the location corresponding to the labeled "tube insert" can assist in assembly and reduce risk of damage and/or unwanted leak path.

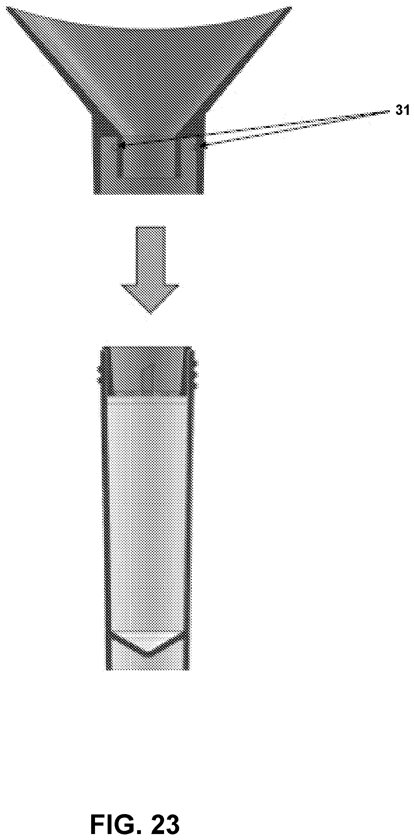

**FIG. 23** illustrates a mouthpiece funnel to tube (and insert) connection. Snap beads **31** are positioned into an upper recess of the mouth funnel. Alternatively, the fit may be a tight clearance fit, with lead-ins on the bottom of the mouth funnel, with added small crush ribs vertically within the inner column (in direction of pull) in order to facilitate a snug fit.

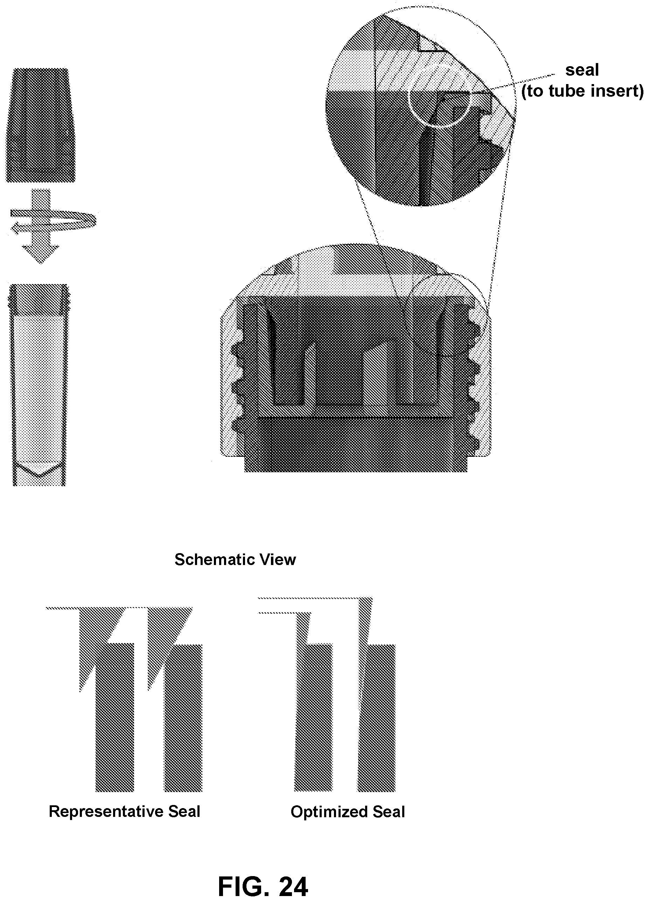

**FIG. 24** illustrates a reagent closure to 5 mL tube (and insert) connection. To ensure a user-applied torque creates a sufficient and reliable seal, a plug seal approach may be used, with a shallower angle of attack (less than 5°), as summarized in the bottom panels.

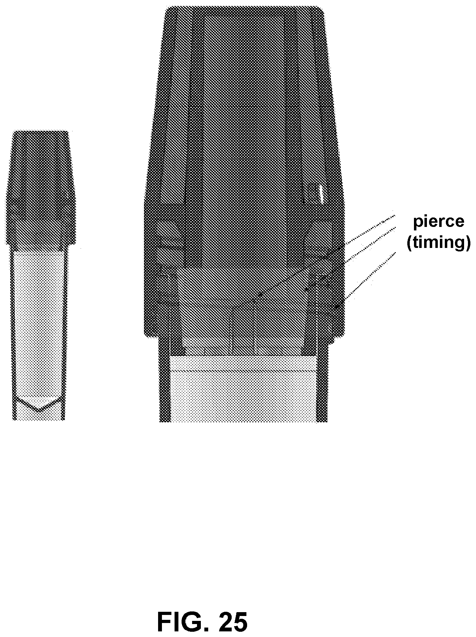

**FIG. 25** illustrates considerations for piercing of the reservoir membrane to the reagent in the integrated reservoir. Preferably, at least one full thread is engaged before piercing.

Additional aspects may include internal leading edge on the collection tube that can be modified and controlled for a best seal and fit. Volume indicators can be positioned to visually indicate sample volume. The collection tube and associated components may be configured in a straight-pull configuration. The mouthpiece may have a rounded edge for user comfort. The cap may have dimensions, such as diameter and height, corresponding to standard caps that are associated with conventional collection tubes. The tube insert and piercing element are configured to avoid damage during use. **FIG. 26** illustrates some embodiments to minimize excessive flex and breakage, including by connecting a plurality of piercing elements, such as by a ring connector that connects a piercing member of the tube insert with at least one or more adjacent piercing members. In this manner, uncontrolled and unwanted movement of the piercing end is avoided, thereby providing more reliable membrane piercing.

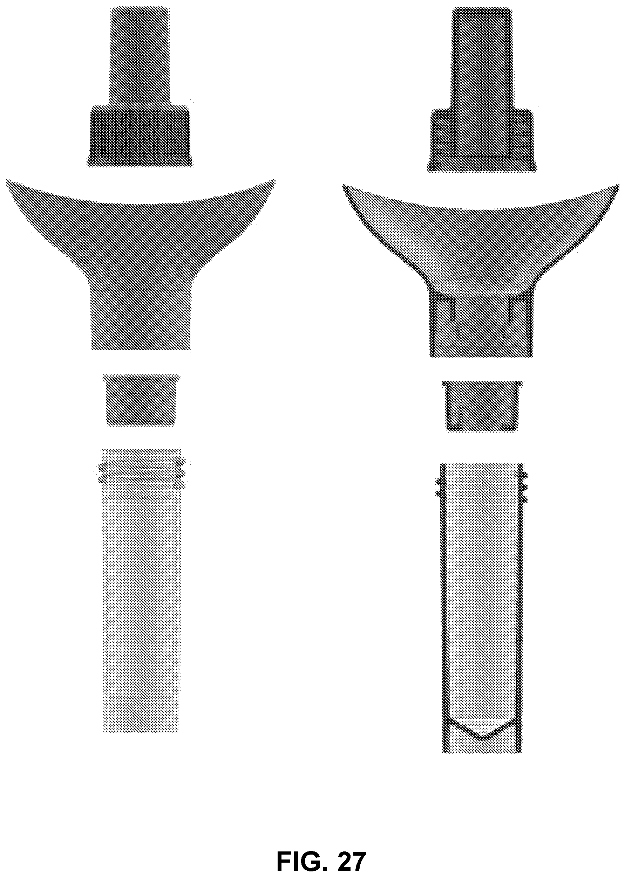

**FIG. 27** illustrates a representative device.

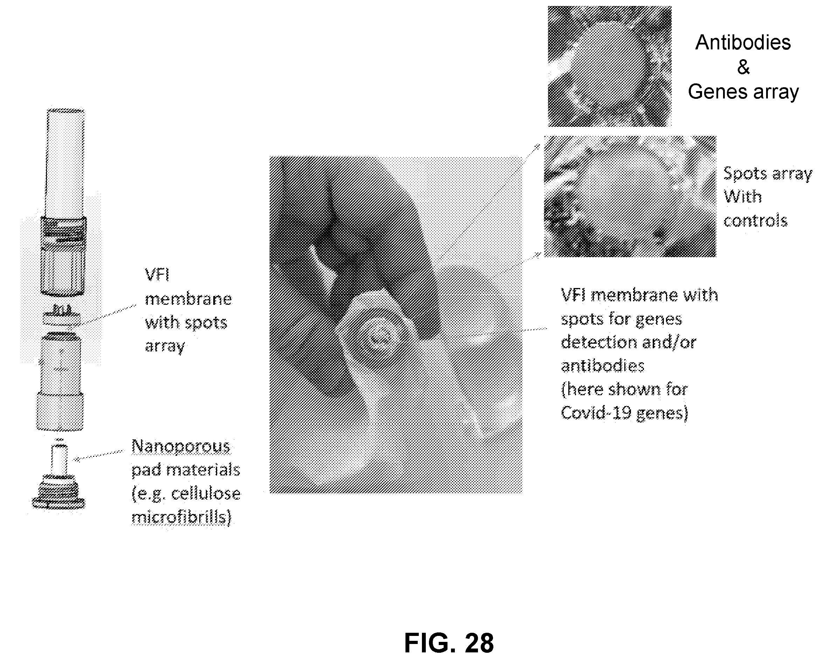

**FIG. 28** illustrates a VFI membrane and pad materials for a biomarker detection assay.

### EXAMPLE 5: Saliva Collection and PCR Testing

According to an embodiment, the methods and devices of the present disclosure can be evaluated for the detection of pathogens. In particular, an exemplary device of the present disclosure can be used to collect a sample for a COVID-19 PCR test for the detection of N1, N3 and RNAseP in individuals at risk for COVID-19. The COVID-19 PCR assay was authorized on August 3, 2020 by EUA201111 for upper respiratory tract samples including nasopharyngeal, oropharyngeal (throat) swab, anterior nasal swab, and mid-turbinate nasal swab samples and nasopharyngeal washes/aspirates or nasal aspirates, and bronchoalveolar lavage.

Below includes data relating to the clinical utility of the COVID-19 PCR test for the detection of SARS-CoV-2 in saliva samples collected using the exemplary device of the present disclosure.

In an embodiment, the exemplary device of the present disclosure allows for a quick, easy, and effective collection strategy. As described herein, the exemplary device is trouble-free (age range 8 - 87 years, all education attainments: at school to PhD) and unaffected by shipping temperatures (-80°C - +40°C). As will be demonstrated below, results from a COVID-19 PCR test based on a sample collected by an exemplary device of the present disclosure generate an accurate determination of SARS-CoV-2, similar to results using nasopharyngeal collection.

To evaluate analytical sensitivity, a limit of detection study (LoD) was performed by spiking biological reagent, or saliva stabilization buffer, with different concentrations of TWIST Bioscience SARS-CoV-2 RNA control (catalogue number MT007544.1, 1,000,000 copies/uL). A total of 3 replicates were tested using the COVID-19 PCR Test per dilution. The viral concentrations ranged from approximately 1 - 1000 copies/uL (data included in **Table 3).**

A standard curve was generated to convert the C_{T} signal to a concentration in copies/mL using a plasmid control from IDT (200,000,000 copies/uL) that was serially diluted from 1 copy/uL to 1000 copies/uL. The LoD from these dilution series was confirmed by spiking 40 individually extracted replicates of negative (control subjects) saliva (all known COVID-19 negative) with the appropriate viral copy number (TWIST Bioscience SARS-CoV-2 RNA control). Twenty-four samples were spiked-in with 15 copies/uL (~1.5xLoD). Ten samples were spiked in with 40-100 copies/uL (~4-10xLoD) and six samples were spiked in with 500-1000 copies/uL (~50-100xLoD). Ten negative controls were also evaluated (no spike-in). Using the COVID-19 PCR test, twenty-four of 24 (24/24) 1.5xLoD replicates were positive, 10/10 (~4-10xLoD) were positive and 6/6 (~10xLoD) were positive. All negative samples were negative (10/10) for the N1 and N3 primers. Summary data for all 40 contrived samples tested are shown below **(Table 4):**

*Clinical Utility Study:* A clinical utility study was performed. The COVID-19 PCR test introduced above was evaluated in a set of 60 COVID-19 tested and known clinical samples. This included 30 verified SARS-CoV-2-positive cases and 30 negative SARS-CoV-2 cases. Nasopharyngeal swabs were collected using the BD Universal Viral transport Kit (BD Catalogue #220529) and stored in UTM for evaluation of viral mRNA using the CDC EUA test (CDC 2019-nCoV Real-Time RT-PCR Diagnostic Panel, unmodified). Matched saliva was collected at the same time point using the exemplary device of the present disclosure for evaluation using the COVID-19 PCR test.

Sample types (nasopharyngeal) were orthogonally compared to the Roche 6800 test (cobas^{®} SARS-CoV-2 test output: positive or negative). Summary data for all 60 clinical samples from each of the two collection types are shown below **(Table 5),** wherein the CDC detection rate is determined by the CDC SARS-CoV-2 Test (N1/N2: CDC 2019-nCoV Real-Time RT-PCR Diagnostic Panel).

Agreement displayed between the results from implementation of the exemplary device of the present disclosure and the CDC assay in matched nasopharyngeal samples is included in **Table 6,** with a two-sided 95% score confidence interval.

The positive percent agreement was 100% (95% confidence interval: 88.4-100.0%). The negative percent agreement was 100% (95% CI: 88.4-100.0%). The accuracy was 100% (95% CI: 94.0-100.0%). The mean C_{T} values for each of the target genes is included in **Table 7,** where * indicates only the CDC assay, ** indicates only an assay based on the methods of the present disclosure, and the 'SARS-CoV-2 RT-PCR Assay' is based on the methods of the present disclosure.

The assay performed based on methods of the present disclosure exhibited similar metrics for SARS-CoV-2 viral detection in saliva as did the CDC assay. Twenty (67%) of 30 nasopharyngeal samples exhibited C_{T} for N1 >35. This identifies these are "low viral titer" samples. The mean C_{T} values for this sub-cohort of clinical samples is included in **Table 8,** where * indicates only the CDC assay, ** indicates only an assay based on the methods of the present disclosure, and the 'SARS-CoV-2 RT-PCR Assay' is based on the methods of the present disclosure.

The assay performed based on the methods of the present disclosure using saliva identified all "low viral titer" nasopharyngeal samples.

*Orthogonal Testing Study:* To perform an orthogonal testing study, the 60 nasopharyngeal samples were also evaluated using the Roche 6800 test (cobas^{®} SARS-CoV-2 test output: positive or negative) as an orthogonal validation test for the assay performed based on the methods of the present disclosure **(Table 9),** where * indicates the sample status was determined by the 2019-nCoV- CDC EUA, ** indicates the detection rate was determined on the basis of the cobas^{®} SARS-CoV-2 test (Roche 6800), ^{#} indicates an *n* of 4 COVID-19 positive samples (by CDC) were identified as positive using an assay based on the methods of the present disclosure but were determined as negative using the Roche test, and 'saliva' indicates the detection rate was determined on the basis of the methods of the present disclosure.

The agreement between the assay in saliva and the Roche test in nasopharyngeal samples is included in **Table 10,** where ¹ is a two-sided 95% score confidence interval.

A Discordant analysis was performed on the 4 false negative results using the CDC EUA assay as authorized (unmodified). All four false negative were also positive by the CDC EUA assay. Results using the COVID-19 PCR test based on the methods of the present disclosure identifies that the test is accurate and effective for the detection of SARS-CoV-2 in saliva collected using an exemplary device of the present disclosure.

*Temperature Testing (ISTA 7D 2007-based shipping) Studies:* Forty-eight saliva samples were evaluated in two separate temperature studies. Samples were collected into an exemplary device of the present disclosure, the exemplary device including a biological reagent, or stabilization buffer.

In a first study (Study 1), per ISTA 7D 2007 "Summer Profile", a total of 48 samples including 23 samples at 1-2xLoD, 5 samples at 2-5xLoD, 10 samples at 5-10xLoD and 10 controls were evaluated over a 56-hour period under variable temperatures (+22°C to +40°C). The temperatures and times for summer profile are included in **Table 11.**

The acceptance criteria were: (a) Low Positive Samples: ≥95% agreement with expected results, (b) High Positive Samples: 100% agreement with expected results, and (c) Negative Samples: 100% agreement with expected results. The results of pre-temperature testing is included in **Table 12,** wherein UD indicates an undetermined value.

All spike-in samples were detected (38/38, 100%) and all controls (10/10 no spike-in) were negative for SARS-CoV-2 detection. The results of the summer temperature test is included in **Table 13,** where UD indicates an undetermined value.

Thirty-seven of 38 (97%) spike-in samples were detected in the summer profile group. Twenty-two of 23 (96%) of 1-2xLoD were detected, and all samples 2-10xLoD were detected. All controls (no spike-in) were negative for SARS-CoV-2 detection. The results identify that the criteria for summer transportation are met by the biological reagent, or saliva stabilization buffer, of the present disclosure.

In a second study (Study 2), for the winter profile, various temperature ranges were evaluated **(Table 14).**

The results of the winter temperature test is included in **Table 15,** where UD indicates an undetermined result.

All spike-in samples were detected (38/38, 100%) and all controls (10/10 no spike-in) were negative for SARS-CoV-2 detection. The results identify that the criteria for winter transportation met by the biological reagent, or saliva stabilization buffer, of the present disclosure.

In summer, it can be appreciated that saliva is an effective source for SARS-CoV-2 detection. The LoD according to methods of the present disclosure is 10 viral copies. The clinical study (*n*=60, matched saliva and nasopharyngeal samples) demonstrates 100% concordance between saliva and nasopharyngeal samples even at "low" viral titers (C_{T}>35), where the positive % agreement is **100%** (95%: 88-100%) and the negative percent agreement: **100%** (95%: 88-100%). The temperature studies (*n*=48, LoD 1.5-10x) identify no impact of heat or cold weather.

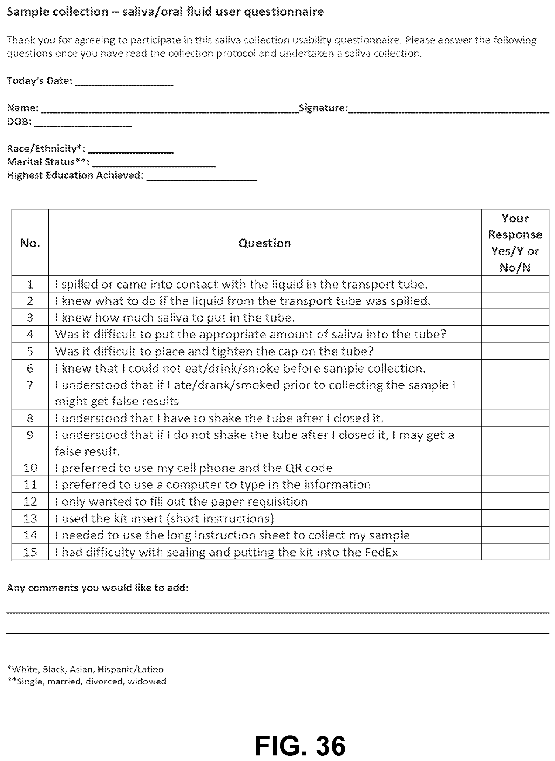

*Usability*/*Human Factors Assessment for the Saliva Collection:* Two studies were performed to evaluate a saliva collection protocol according to methods of the present disclosure. The first study (*n=*122) was undertaken in an industrial setting. The second study (*n*=33) was undertaken in a school setting. Each participant was provided with a kit (including instructions), based on the methods and devices of the present disclosure, and a questionnaire **(****FIG. 36****).** Collection was independently observed. Follow-up calls were made if any questions were flagged or to respond to any comments.

The demographics of the participants is included in **Table 16** and **Table 17.**

The results of the questionnaire are included in **Table 18** and **Table 19,** where * denotes a participant that indicated they had difficulty "making" saliva and drank water. For **Table 19,** 3 participants required parental assistance in order to complete the sample collection.

The results identified that participants of all ages (ranging 5-75), both genders (male/female), and a range of educational achievements (at school to post-graduate degree) had no significant difficulties using the device of the present disclosure (i.e., saliva collection tube), including following instructions and physically collecting the sample.

Moreover, the following observations were made. First, children under 10 years of age require adult supervision. Online data collection (either QR code or typing into a website) was favored by the majority (134/155 = 86.4%). Online data collection was associated with younger individuals (median age: 40 [range: 5-75]. Paper requisitions were associated with older individuals (median age: 58 [range: 30-72]. The shorter kit insert was used in all cases. There were no concerns regarding collection and send-out. Only 1 (0.6%) failure was identified which was related to water ingestion immediately prior to collection. For this one, the RNAseP was greater than 36, which is consistent with a diluted saliva collection. At follow-up, this individual admitted drinking water. She was provided with a second kit and collected the sample appropriately.

*Child Safety Study:* A child safety study was performed to determine whether a sample kit based on the methods and devices of the present disclosure is safe for children.

As background, a COVID-19 PCR Saliva Collection Kit, based on the methods and devices of the present disclosure, has been FDA-EUA authorized (201111, dated: 10/20/2020) for at home collection of saliva from children under a prescription-based setting. Sample collection from children aged 5-13 years is per direct adult collection. Sample collection for 14-17 years is per direct adult supervision.

To demonstrate child-safety feasibility, a prospective, observational study, evaluating the child-safety aspects of the collection cap (which contains a guanidine-based stabilization buffer) and the post-collection tube (capped saliva-collected sample), was performed. The goals of the study were to evaluate whether regular play had an impact on the aluminum seal of the cap, the cap structure, and sealing at the cap/tube connection, evaluate whether children could break the aluminum seal of the cap and/or break the cap, and evaluate whether children could open the tube once a sample was collected.

Individual subject demographics are included in **Table 20,** where F is female, M is male, A is Asian, B is black, C is Caucasian, H is Hispanic/Latino, and NHL is non-Hispanic/Latino.

A total of 12 children were observed with ages ranging between 5-17 years (median: 10 years), including 7 girls and 5 boys. Race, ethnicity and school grade spanned all demographics.

For "Regular play" observations, children were provided a standard sealed cap and observed for up to 60 minutes. Most children spent a median of 20 minutes playing with the object. Play ranged from throwing and kicking it to spinning the cap. Girls tended to incorporate the object into their imaginary play (e.g., use it as a prop - e.g., a cup - in their play).

Most children threw and kicked the cap, with the majority also spinning it.

For the "breakage" observations, children were asked to remove the foil with their fingers/fingernails or to break the cap by standing on it. The same caps were used as those used in the play portion of the observation. Most children used their nails to try and unpeel the seal or used a variety of options to break the cap including pressing down with their fingernail (thumb, index or middle finger).

The individual results are included in **Table 21,** where NE is no effect, NI is no impact, NOD is not observed/not done by the child, and * denotes a child that was observed a second time. They were asked to do the same things (play, break) with the same outcome - no impact on the cap or the cap/tube connection.

The median observation time was 20 minutes (range: 11-57 minutes).

Individual play/breakage observations included that the foil has some give so it can be pushed inwards without breaking, the foil is recessed too deeply and even little fingers/fingernails could not access it to unpeel the foil, all caps (after regular play and breakage testing) could be screwed onto the tube and were functional (i.e., could dispense the liquid into the tube).

In summary, regular play had no impact on the aluminum seal or cap structure itself. Caps functioned normally and could be screwed onto tubes. Children were unable to unpeel the aluminum seal or break the foil with their fingernail. They could not break the cap. Further, a tightened cap (post-collection) requires significant force to open it. Children could not open the cap after it was tightened.

For post-collection cap removal observations, the same group was provided with capped saliva collection tubes. They were asked to remove the caps using any means. Children were observed using a variety of grips including thumb/first digit, thumb/multiple digits, power grip, two hands, single twist (holding the tube with one hand, using the other hand to untwist), and double twisting (one hand on tube, the other on the cap and untwisting - working in opposite directions). Children were encouraged to use multiple approaches.

The individual results are included in **Table 22,** where NE is no effect, NI is no impact, NOD is not observed/not done by the child, and * denotes children that were observed a second time. They were asked to unscrew the cap on a second occasion. The result was the same outcome - no impact on the cap/tube connection.

The median time observed to unscrew the cap was 5.5 minutes (range: 4-12 minutes). Ultimately, all capped tubes were returned to the laboratory for the technician to remove. In all instances, the caps cannot be opened using thumb and first/second fingers. A power grip was required with a double twist, to open the caps.

A summary of observations is included in **Table 23,** where * denotes cases of cap breakage, ** denotes cases of cap breakage, ^{†} indicates whether the cap could be screwed onto a tube after play/breakage attempts, and ^{‡} indicates whether the cap could be opened by a child after it was screwed onto a tube.

Based on these observations, it can be concluded that the cap and the capped collection tube is difficult to open and that the 10 children aged 5-13 years were not able to break the seal, break the cap during play or intentionally and they cannot open the sample collection system once closed.

### STATEMENTS REGARDING VARIATIONS

The terms and expressions which have been employed herein are used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments, exemplary embodiments and optional features, modification and variation of the concepts herein disclosed may be resorted to by those skilled in the art, and that such modifications and variations are considered to be within the scope of this invention as defined by the appended claims. The specific embodiments provided herein are examples of useful embodiments of the present invention and it will be apparent to one skilled in the art that the present invention may be carried out using a large number of variations of the devices, device components, methods steps set forth in the present description. As will be obvious to one of skill in the art, methods and devices useful for the present methods can include a large number of optional composition and processing elements and steps.

When a group of substituents is disclosed herein, it is understood that all individual members of that group and all subgroups, are disclosed separately. When a Markush group or other grouping is used herein, all individual members of the group and all combinations and sub-combinations possible of the group are intended to be individually included in the disclosure.

Every formulation or combination of components described or exemplified herein can be used to practice the invention, unless otherwise stated.

Whenever a range is given in the specification, for example, a size range, number range, a time range, or a composition or concentration range, all intermediate ranges and subranges, as well as all individual values included in the ranges given are intended to be included in the disclosure. It will be understood that any subranges or individual values in a range or subrange that are included in the description herein can be excluded from the claims herein.

All patents and publications mentioned in the specification are indicative of the levels of skill of those skilled in the art to which the invention pertains.

As used herein, "comprising" is synonymous with "including," "containing," or "characterized by," and is inclusive or open-ended and does not exclude additional, unrecited elements or method steps. As used herein, "consisting of" excludes any element, step, or ingredient not specified in the claim element. As used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms. The invention illustratively described herein suitably may be practiced in the absence of any element or elements, limitation or limitations which is not specifically disclosed herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

### TABLES:

**Table 1. Test conditions for cap and capped tube combination.**

| **Time (hrs)** | **Pressure (kPa)** | **Cap** | **Cap+Tube Combination** |
|---|---|---|---|
| 0.5 | 0, 25, 50, 75, 95 | N=3 | N=3 |
| 1 | 0, 25, 50, 75, 95 | N=3 | N=3 |
| 2 | 0, 25, 50, 75, 95 | N=3 | N=3 |
| 4 | 0, 25, 50, 75, 95 | N=3 | N=3 |
| 8 | 0, 25, 50, 75, 95 | N=3 | N=3 |
| 16 | 0, 25, 50, 75, 95 | N=3 | N=3 |
| 24 | 0, 25, 50, 75, 95 | N=3 | N=3 |
| 48 | 0, 25, 50, 75, 95 | N=3 | N=3 |

**Table 2: Pressure test results for cap and cap-collection tube combination.**

| **No.** | **Experiment type** | **Cap Results** | **% Pass rate** | **Cap+Tube Combination** | **% Pass rate** |
|---|---|---|---|---|---|
| 1 | Time: 0.5hr/ Pressure: 0 kPa | 3/3 pass | 100 | 3/3 pass | 100 |
| | Time: 0.5hr/ Pressure: 25 kPa | 3/3 pass | 100 | 3/3 pass | 100 |
| | Time: 0.5hr/ Pressure: 50 kPa | 3/3 pass | 100 | 3/3 pass | 100 |
| | Time: 0.5hr/ Pressure: 75 kPa | 3/3 pass | 100 | 3/3 pass | 100 |
| | Time: 0.5hr/ Pressure: 95 kPa | 3/3 pass | 100 | 3/3 pass | 100 |
| 2 | 1hr/0kPa | 3/3 pass | 100 | 3/3 pass | 100 |
| | 1hr/25kPa | 3/3 pass | 100 | 3/3 pass | 100 |
| | 1hr/50kPa | 3/3 pass | 100 | 3/3 pass | 100 |
| | 1hr/75kPa | 3/3 pass | 100 | 3/3 pass | 100 |
| | 1hr/95kPa | 3/3 pass | 100 | 3/3 pass | 100 |
| 3 | 2hr/0kPa | 3/3 pass | 100 | 3/3 pass | 100 |
| | 2hr/25kPa | 3/3 pass | 100 | 3/3 pass | 100 |
| | 2hr/50kPa | 3/3 pass | 100 | 3/3 pass | 100 |
| | 2hr/75kPa | 3/3 pass | 100 | 3/3 pass | 100 |
| | 2hr/95kPa | 3/3 pass | 100 | 3/3 pass | 100 |
| 4 | 1hr/0kPa | 3/3 pass | 100 | 3/3 pass | 100 |
| | 4hr/25kPa | 3/3 pass | 100 | 3/3 pass | 100 |
| | 4hr/50kPa | 3/3 pass | 100 | 3/3 pass | 100 |
| | 4hr/75kPa | 3/3 pass | 100 | 3/3 pass | 100 |
| | 4hr/95kPa | 3/3 pass | 100 | 3/3 pass | 100 |
| 5 | 8hr/0kPa | 3/3 pass | 100 | 3/3 pass | 100 |
| | 8hr/25kPa | 3/3 pass | 100 | 3/3 pass | 100 |
| | 8hr/50kPa | 3/3 pass | 100 | 3/3 pass | 100 |
| | 8hr/75kPa | 3/3 pass | 100 | 3/3 pass | 100 |
| | 8hr/95kPa | 3/3 pass | 100 | 3/3 pass | 100 |
| 6 | 16hr/0kPa | 3/3 pass | 100 | 3/3 pass | 100 |
| | 16hr/25kPa | 3/3 pass | 100 | 3/3 pass | 100 |
| | 16hr/50kPa | 3/3 pass | 100 | 3/3 pass | 100 |
| | 16hr/75kPa | 3/3 pass | 100 | 3/3 pass | 100 |
| | 16hr/95kPa | 3/3 pass | 100 | 3/3 pass | 100 |
| 7 | 24hr/0kPa | 3/3 pass | 100 | 3/3 pass | 100 |
| | 24hr/25kPa | 3/3 pass | 100 | 3/3 pass | 100 |
| | 24hr/50kPa | 3/3 pass | 100 | 3/3 pass | 100 |
| | 24hr/75kPa | 3/3 pass | 100 | 3/3 pass | 100 |
| | 24hr/95kPa | 3/3 pass | 100 | 3/3 pass | 100 |
| 8 | 48hr/0kPa | 3/3 pass | 100 | 3/3 pass | 100 |
| | 48hr/25kPa | 3/3 pass | 100 | 3/3 pass | 100 |
| | 48hr/50kPa | 3/3 pass | 100 | 3/3 pass | 100 |
| | 48hr/75kPa | 3/3 pass | 100 | 3/3 pass | 100 |
| | 48hr/95kPa | 2/3 pass | 67 | 3/3 pass | 100 |

**Table 3: C_{T} values for primers N1 and N3 in TWIST LoD spike-in samples.**

| | **C_{T} Values** | | | | **Detection Rate** | |
|---|---|---|---|---|---|---|
| Virus | **N1** | | **N3** | | **N1** | **N3** |
| copies/uL | **Mean** | **SD** | **Mean** | **SD** | | |
| **1** | 39.27 | 0.97 | 38.73 | 0.22 | 2/3 | 2/3 |
| **10** | 36.76 | 0.59 | 37.34 | 0.74 | 3/3 | 3/3 |
| **100** | 36.18 | 0.21 | 35.80 | 0.17 | 3/3 | 3/3 |
| **1000** | 32.24 | 0.28 | 32.45 | 0.46 | 3/3 | 3/3 |

**Table 4: Summary data of 40 contrived and 10 control samples. (*UD = undetermined)**

| **SARS-CoV-2 concentration** | **No.** | **Detection rate** | **N1 C_{T} (mean)** | | **N3 C_{T} (mean)** | | **RNAseP C_{T} (mean)** |
|---|---|---|---|---|---|---|---|
| 1.5x LoD (15 copies/uL) | 24 | 24 | 36.75 | | 36.77 | | 30.14 |
| 3-10X LoD (40-100 copies/uL) | 10 | 10 | 36.06 | | 36.6 | | 29.63 |
| 50-100X LoD (500-1000 copies/uL) | 6 | 6 | 33.83 | | 33.51 | | 30.06 |
| Negative | 10 | 10 | UD* | | UD | | 29.4 |

**Table 5: Clinical evaluation summary data of 60 clinical samples (vs. CDC)**

| **SARS-CoV-2 sample status** | **Detection rate (CDC)* [Nasopharyngeal swab]** | **Detection rate [Saliva]** |
|---|---|---|
| Negative (*n*=30) | 30 | 30 |
| Positive (*n*=30) | 30 | 30 |

**Table 6:**

| | | **CDC 2019-nCoV Real-Time RT-PCR Diagnostic Panel Authorized Assay - Comparator** | | |
|---|---|---|---|---|
| | | **Positive** | **Negative** | **Total** |
| **SARS-CoV-2 RT-PCR Assay** | **Positive** | 30 | 0 | 30 |
| | **Negative** | 0 | 30 | 30 |
| | **Total** | 30 | 30 | 60 |
| **Positive Agreement** | | 100.00% (30/30); 88.43-100.0% ¹ | | |
| **Negative Agreement** | | 100% (30/30); 88.43-100.0% | | |

**Table 7: Summary data of 30 positive clinical samples.**

| **Assay** | **No.** | **Detection rate** | **N1 C_{T} (mean)** | **N2 C_{T} (mean)*** | **N3 C_{T} (mean)**** | **RNAseP C_{T} (mean)** |
|---|---|---|---|---|---|---|
| **SARS-CoV-2 RT-PCR Assay** | 30 | 30 | 33.41 | - | 33.06 | 30.86 |
| **CDC 2019-nCoV Real-Time RT-PCR Diagnostic Panel Authorized Assay - Comparator** | 30 | 30 | 33.14 | 32.45 | - | 30.46 |

**Table 8: Summary data of 20 "low viral titer" clinical samples.**

| **Assay** | **No.** | **Detection rate** | **N1 C_{T} (mean)** | | **N2 C_{T} (mean)*** | | **N3 C_{T} (mean)**** |
|---|---|---|---|---|---|---|---|
| **SARS-CoV-2 RT-PCR Assay** | 20 | 20 | 37.04 | | - | | 36.29 |
| **CDC 2019-nCoV Real-Time RT-PCR Diagnostic Panel Authorized Assay - Comparator** | 20 | 20 | 36.7 | | 35.89 | | - |

**Table 9: Clinical evaluation summary data of 60 clinical samples (saliva vs. Roche [nasopharyngeal])**

| **SARS-CoV-2 sample status*** | **Detection rate (saliva)** | **Detection rate (Roche)**** |
|---|---|---|
| Negative | 30 | 30 |
| Positive | 30 | 26^{#} |

**Table 10:**

| | | **Cobas SARS-CoV-2 Test (Roche 6800) Authorized Assay - Comparator** | | |
|---|---|---|---|---|
| | | **Positive** | **Negative** | **Total** |
| **Wren Laboratories SARS-CoV-2 RT-PCR Assay** | **Positive** | 26 | 4^{a} | 30 |
| | **Negative** | 0 | 30 | 30 |
| | **Total** | 26 | 34 | 60 |
| **Positive Agreement** | | 86.67% (26/30); 69.28-96.24% ¹ | | |
| **Negative Agreement** | | 100% (30/30); 83.80-98.15% | | |

**Table 11:**

| **Temperature** | **Cycle Period** | **Cycle Period Hours** | **Total Time Hours** |
|---|---|---|---|
| 40°C | 1 | 8 | 8 |
| 22°C | 2 | 4 | 12 |
| 40°C | 3 | 2 | 14 |
| 30°C | 4 | 36 | 50 |
| 40°C | 5 | 6 | 56 |

**Table 12: Pre-temperature testing results.**

| **SARS-CoV-2 concentration** | **Number of samples** | **Detection rate** | **N1 C_{T} (mean)** | **N3 C_{T} (mean)** | **RNAseP C_{T} (mean)** |
|---|---|---|---|---|---|
| 1-2X LoD | 23 | 23 | 36.27 | 35.68 | 30.08 |
| 2-5xLoD | 5 | 5 | 35.36 | 35.04 | 29.83 |
| 5-10x LoD | 10 | 10 | 33.61 | 33.09 | 29.22 |
| Negative (asymptomatic) | 10 | 10 | UD | UD | 29.47 |

**Table 13: Summer testing results.**

| **SARS-CoV-2 concentration** | **Number of samples** | **Detection rate** | **N1 C_{T} (mean)** | **N3 C_{T} (mean)** | **RNAseP C_{T} (mean)** |
|---|---|---|---|---|---|
| 1-2X LoD | 23 | 22 | 36.51 | 35.81 | 30.04 |
| 2-5xLoD | 5 | 5 | 36.64 | 35.93 | 29.86 |
| 5-10x LoD | 10 | 10 | 34.54 | 34.28 | 28.86 |
| Negative (asymptomatic) | 10 | 10 | UD | UD | 29.27 |

**Table 14: Winter profile.**

| **Temperature** | **Cycle Period** | **Cycle Period Hours** | **Total Time Hours** |
|---|---|---|---|
| -10°C | 1 | 8 | 8 |
| 18°C | 2 | 4 | 12 |
| -10°C | 3 | 2 | 14 |
| 10°C | 4 | 36 | 50 |
| -10°C | 5 | 6 | 56 |

**Table 15: Winter testing results.**

| **SARS-CoV-2 concentration** | **Number of samples** | **Detection rate** | **N1 C_{T} (mean)** | **N3 C_{T} (mean)** | **RNAseP C_{T} (mean)** |
|---|---|---|---|---|---|
| 1-2X LoD | 23 | 23 | 34.88 | 33.17 | 27.41 |
| 2-5xLoD | 5 | 5 | 35.72 | 36.25 | 29.19 |
| 5-10x LoD | 10 | 10 | 34.62 | 34.10 | 28.62 |
| Negative (asymptomatic) | 10 | 10 | UD | UD | 29.02 |

**Table 16: Human Factor Assessment - Demographics - Study One (n=122)**

| **Age** | **Gender** | **Education** | |
|---|---|---|---|
| 46 (17 - 75) | M: 115 (94%) | At school | 1 (1%) |
| Median (range) | F: 7 (6%) | Diploma | 94 (77%) |
| | | Undergraduate | 22 (18%) |
| | | Postgraduate | 5 (4%) |

**Table 17: Human Factor Assessment - Demographics - Study Two (n=33)**

| **Age** | **Gender** | **Education** | |
|---|---|---|---|
| 16 (5 - 65) | M: 19 (58%) | At school | 20 (61%) |
| Median (range) | F: 14 (42%) | Diploma | 8 (24%) |
| | | Undergraduate | 0 (0%) |
| | | Postgraduate | 5 (15%) |

**Table 18: Human Factor Assessment - Results of Questionnaire - Study One (n=122)**

| **Question #** | **Expected Answer** | **Received - Yes** | **Received - No** |
|---|---|---|---|
| 1 | N | 0 | 122 |
| 2 | Y | 122 | 0 |
| 3 | Y | 122 | 0 |
| 4 | N | 1* | 121 |
| 5 | N | 0 | 122 |
| 6 | Y | 122 | 0 |
| 7 | Y | 122 | 0 |
| 8 | Y | 122 | 0 |
| 9 | Y | 122 | 0 |
| 10 | Y | 78 | 44 |
| 11 | N | 24 | 98 |
| 12 | N | 20 | 102 |
| 13 | Y | 122 | 0 |
| 14 | N | 0 | 122 |
| 15 | N | 0 | 122 |

**Table 19: Human Factor Assessment - Results of Questionnaire- Study Two (n=33)**

| **Question #** | **Expected Answer** | **Received - Yes** | **Received - No** |
|---|---|---|---|
| 1 | N | 0 | 33 |
| 2 | Y | 33 | 0 |
| 3 | Y | 33 | 0 |
| 4 | N | 0 | 33 |
| 5 | N | 0 | 33 |
| 6 | Y | 33 | 0 |
| 7 | Y | 33 | 0 |
| 8 | Y | 33 | 0 |
| 9 | Y | 33 | 0 |
| 10 | Y | 27 | 6 |
| 11 | N | 5 | 28 |
| 12 | N | 1 | 32 |
| 13 | Y | 33 | 0 |
| 14 | N | 0 | 33 |
| 15 | N | 0 | 33 |

**Table 20:**

| **No.** | **Age (Years)** | **Gender** | **Race** | **Ethnicity** | **School Grade** |
|---|---|---|---|---|---|
| 1 | 5 | F | B | NHL | K |
| 2 | 6 | F | C | NHL | 1 |
| 3 | 7 | M | C | HL | 2 |
| 4 | 8 | M | C | HL | 2 |
| 5 | 9 | F | B | NHL | 4 |
| 6 | 10 | M | C | NHL | 4 |
| 7 | 10 | F | C | NHL | 5 |
| 8 | 11 | F | C | NHL | 5 |
| 9 | 12 | M | B | NHL | 6 |
| 10 | 13 | F | A | NHL | 7 |
| 11 | 16 | F | C | HL | 10 |
| 12 | 17 | M | C | NHL | 11 |

**Table 21:**

| **No.** | **Total Time under observation** | **Regular Play** | | | **Cap Breakage** | | | **Cap/Tube connection** |
|---|---|---|---|---|---|---|---|---|
| | | Throw and/or Kick | Spinning | Imaginary play | Unpeeling foil | Break foil with fingernail | Breaking cap* | |
| 1* | 37 | NE | NE | NE | NE | NE | NE | NI |
| 2 | 41 | NE | NE | NE | NE | NE | NE | NI |
| 3 | 17 | NE | NE | NOD | NE | NE | NE | NI |
| 4 | 15 | NE | NE | NOD | NE | NE | NE | NI |
| 5 | 41 | NE | NE | NE | NE | NE | NE | NI |
| 6* | 57 | NE | NE | NE | NE | NE | NE | NI |
| 7 | 23 | NE | NOD | NOD | NE | NE | NE | NI |
| 8 | 19 | NE | NE | NOD | NE | NE | NE | NI |
| 9 | 11 | NE | NOD | NOD | NE | NE | NE | NI |
| 10 | 21 | NE | NE | NOD | NE | NE | NE | NI |
| 11 | 18 | NE | NOD | NOD | NE | NE | NE | NI |
| 12* | 16 | NE | NE | NOD | NE | NE | NE | NI |

**Table 22**

| **No.** | **Total Time under observation** | **Unscrew approach** | | | | | **Cap/Tube connection** |
|---|---|---|---|---|---|---|---|
| | | Thumb/1^{st} digit | Thumb Multiple | Power grip | Single twisting | Double twisting | |
| 1* | 6 | NE | NE | NE | NE | NE | NI |
| 2 | 5 | NE | NE | NE | NE | NOD | NI |
| 3 | 4 | NE | NE | NE | NE | NOD | NI |
| 4 | 5 | NE | NE | NE | NE | NE | NI |
| 5 | 6 | NE | NE | NE | NE | NE | NI |
| 6* | 12 | NE | NE | NE | NE | NE | NI |
| 7 | 5 | NE | NE | NE | NE | NE | NI |
| 8 | 6 | NE | NE | NE | NE | NE | NI |
| 9 | 4 | NE | NE | NE | NE | NOD | NI |
| 10 | 8 | NE | NE | NE | NE | NOD | NI |
| 11 | 6 | NE | NE | NE | NE | NOD | NI |
| 12* | 5 | NE | NE | NE | NE | NOD | NI |

**Table 23**

| | **PLAY*** | **BREAKAGE**** | **CAP INTEGRITY^{†}** | **CAP REOPENING^{‡}** |
|---|---|---|---|---|
| **Children 5-13 vears (*n*=10)** | 0/10 (0%) | 0/10 (0%) | 10/10 (100%) | 0/10 (0%) |
| **Children 14-17 vears (*n*=2)** | 0/2 (0%) | 0/2 (0%) | 2/2 (100%) | 0/2 (0%) |
| **TOTAL** | 0/12 (0%) | 0/12 (0%) | 12/12 (100%) | 0/12 (0%) |

**Table 24: Exemplary Saliva Stabilization Buffer - for use with Saliva collection:**

| **Chemical** | **(CAS #)** | **Purity specification** | **% (w/w or w/v)** | **Concentration (mg/ml)** | **Total amount (mg) in 1000 ml solution** |
|---|---|---|---|---|---|
| Guanidine Hydrochloride | 50-01-1 | ≥99% | | 4.5M | 429.88g |
| Sodium citrate | 6132-04-3 | ≥99% | | 120mM | 35.3g |
| Citric acid | 77-92-9 | 99% | | 80mM | 15.36g |
| EDTA | 60-00-4 | ≥99% | | 20mM | 66.6mL of 0.3M stock |
| Triton X-100 | 9002-93-1 | NA | 0.1% (v/v) | | 1000uL |
| FD&C Red No. 40 | Allura Red AC, E129 | NA | 0.1% (v/v) | | 1000uL |
| UltraPure^{™} DNase/RNase-Free Distilled Water | q.s. to 1000 mL | | | | |

| | | | | | |
|---|---|---|---|---|---|
| All reagents commercially available, such as from SIGMA | | | | | |

**Table 25: List of antibodies against SARS-CoV-2 S protein (Uniprot P0DTC2) and with known sequence**

| **ABCD** | **Ab Name** | **PMID/DOI** | **PDB** | **Epitope** | **Comments** |
|---|---|---|---|---|---|
| AI334 | CR302 2 | 16796401, 32245784 | 6W41 | S1 (356-504) | Isolated from CoV-1 patient; defined 3D structure |
| AQ806 | VHH-72 | 32375025 | 6WAQ | S1 (RBD/ 355-366) | Nanobody raised against CoV-1; defined 3D structure |
| AR209-AR271 | Sb#XX X (63 Abs) | 10.1101/2020.04.16.0454 19 | | S1 (RBD/ 330-526) | Nanobodies (top-performers: AR222, AR223, AR224, AR248, AR249, AR269) |
| AS273 AS274 | B38 H4 | 32404477 | 7BZ5 | S1 (RBD) | Isolated from CoV-2 patient; defined 3D structure |
| AS682-AS725 | (44 Abs) | 32511342 | 6XE1 | | Isolated from CoV-2 patient Only 2 neutralizing and targeting RBD (AS682 and AS708); defined 3D structure |
| AS739 | S309 | 32422645 | 6WPS | S1 (non-RBD) | Isolated from CoV-1 patient; defined 3D structure |
| AS740 AS862 | CB6 CA1 | 32454512 | 7C01 | S1 (RBD) | Isolated from CoV-2 patient; defined 3D structure; prevents infections in monkeys |
| AT085 | P2B-2F6 | 32454513 | 7BWJ | S1 (RBD) | Isolated from CoV-2 patient; defined 3D structure |
| AT086 | NbTyl | 10.1101/2020.06.02.1301 61 | | S1 (RBD) | Nanobody; defined 3D structure (no PDB) |
| AT460-AT542 | (63 Abs) | 10.1101/2020.05.13.0926 19v2 10.1101/2020.05.28.1215 33 | N.A. | S1 (RBD) | Isolated from CoV-2 patients; defined 3D structure for AT483 |
| AT693 | BD23 | 32425270 | 7BYR | S1 (RBD) | Isolated from CoV-2 patient; defined 3D structure |
| AT798 AT799 | W23U ACh W25U ACh | 10.1101/2020.06.09.1379 35 | | | Nanobodies |
| AT800-AT828 | (29 Abs) | 10.1101/2020.06.09.1434 38 | 7C8V, 7C8W, 7CAN | S1 (RBD) | Nanobodies; 3 with defined 3D structure (AT801, AT817, AT828) |
| AT868-AT876 | (9 Abs) | 32540901 | 6XDG | S1 (RBD) | Regeneron Abs; REGN10933+REGN10 987 (AT870+AT869) in clinical trials |
| AT877-AT958 | (82 Abs) | 32540902 | | | Isolated from CoV-2 patients (AT892 and AT916 best neutralizers) |
| AT959 AT960 | CC12.1 CC12.3 | 10.1101/2020.05.11.0886 74v2 | 6XC2 6XC4 | S1 (RBD) | Isolated from CoV-2 patient; defined 3D structure |

## Claims

1. A kit for self-collection and processing of a biofluid that may contain a biological specimen or a biomarker of the biological specimen from a user comprising:
a mouthpiece (30);
a collection tube (40) fluidically connected to the mouthpiece (30);
a tube insert (50) positioned in the collection tube (40), wherein the tube insert (50) comprises:
a piercing end (60);
a cap (70) configured to connect to the collection tube (40) and fluidically seal the collection tube (40) from a surrounding environment, wherein the cap (70) comprises:
an integrated reservoir (71) configured to hold a biological reagent (72) configured for use with the biological specimen in the collection tube (40); a reservoir membrane (73) to contain the biological reagent (72) in the integrated reservoir (71) and to temporarily seal the integrated reservoir (71) from the collection tube (40), wherein the tube insert (50) piercing end (60) is configured to pierce the reservoir membrane (73) and fluidically contact the biofluid in the collection tube (40) and the biological reagent in the integrated reservoir (71), and wherein the reservoir membrane (73) is configured to be removable after the biological reagent (72) is introduced to the biofluid in the collection tube (40); and
a biomarker detection membrane (54) configured to connect to the cap (70) after the reservoir membrane (73) is removed to detect one or more biomarkers in the biofluid.

2. The kit of claim 1, wherein the tube insert further comprises:
a positioning feature (51) to align and position the piercing end (60) at a pre-determined distance (52) from the reservoir membrane (73).

3. The kit of claim 1 or 2, further comprising:
the detection membrane (54) positioned at the bottom of the tube insert (50);
a means (56) for driving fluid flow through the detection membrane (54) to obtain a biological measurement; and
a holder (57) for holding the detection membrane (54).

4. The kit of any of claims 1-3, wherein the reservoir membrane (73) is a re-sealable membrane.

5. The kit of claim 4, wherein the re-sealable membrane comprises:
an interchangeable adhesive layer (74); and
a porous substrate (75) comprising biomarker detectors (76).

6. The kit of claim 5, wherein the biomarker detectors (76) are selected from the group consisting of: polynucleotides, polypeptides, antibodies, nucleic acids, toxins, bacteria, virus, biological vesicles, the device further comprising a plurality of unique biomarker detectors (76) arranged in a microarray (77) on the detection membrane (54) for multiplex detection of biomarkers in a biofluid.

7. The kit of any of claims 1-6, further comprising:
a vertical flow immunoassay (VFI) (100) and/or a lateral flow assay (LFA) integrated with the reservoir membrane (73) or the detection membrane (54), including a detection membrane (54) that is a VFI or LFA membrane comprising one or more biomarker detection elements (76), optionally the biomarker detection elements (76) are antibodies, wherein the vertical flow immunoassay (100) comprises:
a sandwich enzyme-linked immunosorbent assay (ELISA) (101);
a means (56) for driving fluid flow through the detection membrane (54);
wherein the device is configured to reflect presence of the biomarker by a change in optical color at the membrane.

8. The kit of any claims 1-7, wherein the tube insert (50) comprises:
an insert body (150) shaped for insertion into a reservoir (71) of the collection tube (40);
a flange (151) on the insert body (150) configured to be supported by an entrance wall (152) of the collection tube (40);
a bottom surface (58) having a central opening (59); and
wherein the piercing end (60) corresponds to a spike (61) extending from the bottom surface (58) and the piercing end (60) corresponds to a plurality of physically separated spikes (61).

## Patentansprüche

1. Kit zur Selbstsammlung und Verarbeitung eines Biofluids, das eine biologische Probe oder einen Biomarker der biologischen Probe von einem Benutzer enthalten kann, umfassend:
ein Mundstück (30);
ein Sammelrohr (40), das fluidmäßig mit dem Mundstück (30) verbunden ist;
einen Rohreinsatz (50), der in dem Sammelrohr (40) positioniert ist, wobei der Rohreinsatz (50) Folgendes umfasst:
ein Einstichende (60);
eine Kappe (70), die dazu konfiguriert ist, sich mit dem Sammelrohr (40) zu verbinden und das Sammelrohr (40) fluidmäßig gegenüber einer umliegenden Umgebung abzudichten, wobei die Kappe (70) Folgendes umfasst:
einen integrierten Vorratsbehälter (71), der dazu konfiguriert ist, ein biologisches Reagenz (72) aufzunehmen, das zur Verwendung mit der biologischen Probe in dem Sammelrohr (40) konfiguriert ist; eine Vorratsbehältermembran (73) zum Enthalten des biologischen Reagenzes (72) in dem integrierten Vorratsbehälter (71) und zum vorübergehenden Abdichten des integrierten Vorratsbehälters (71) gegen das Sammelrohr (40), wobei das Einstichende (60) des Rohreinsatzes (50) dazu konfiguriert ist, die Vorratsbehältermembran (73) zu durchstechen und das Biofluid in dem Sammelrohr (40) und das biologische Reagenz in dem integrierten Vorratsbehälter (71) fluidmäßig zu kontaktieren, und wobei die Vorratsbehältermembran (73) dazu konfiguriert ist, entfernbar zu sein, nachdem das biologische Reagenz (72) in das Biofluid in dem Sammelrohr (40) eingeführt wurde; und
eine Biomarker-Detektionsmembran (54), die dazu konfiguriert ist, sich mit der Kappe (70), nachdem die Vorratsbehältermembran (73) entfernt wurde, zu verbinden, um einen oder mehrere Biomarker in dem Biofluid nachzuweisen.

2. Kit nach Anspruch 1, wobei der Rohreinsatz ferner Folgendes umfasst:
ein Positionierungsmerkmal (51), um das Einstichende (60) in einem vorbestimmten Abstand (52) von der Vorratsbehältermembran (73) auszurichten und zu positionieren.

3. Kit nach Anspruch 1 oder 2, ferner umfassend:
die Detektionsmembran (54), die an der Unterseite des Rohreinsatzes (50) positioniert ist;
ein Mittel (56) zum Antreiben eines Fluidstroms durch die Detektionsmembran (54), um eine biologische Messung zu erhalten; und
eine Halterung (57) zum Halten der Detektionsmembran (54).

4. Kit nach einem der Ansprüche 1 bis 3, wobei die Vorratsbehältermembran (73) eine wiederverschließbare Membran ist.

5. Kit nach Anspruch 4, wobei die wiederverschließbare Membran Folgendes umfasst:
eine austauschbare Klebstoffschicht (74); und
ein poröses Substrat (75), das Biomarkerdetektoren (76) umfasst.

6. Kit nach Anspruch 5, wobei die Biomarkerdetektoren (76) aus der Gruppe ausgewählt sind, die aus Folgendem besteht: Polynukleotiden, Polypeptiden, Antikörpern, Nukleinsäuren, Toxinen, Bakterien, Viren, biologischen Vesikeln, wobei die Vorrichtung ferner eine Vielzahl von einzigartigen Biomarkerdetektoren (76) umfasst, die in einem Mikroarray (77) auf der Detektionsmembran (54) zur Multiplex-Detektion von Biomarkern in einem Biofluid angeordnet sind.

7. Kit nach einem der Ansprüche 1 bis 6, ferner umfassend:
einen Vertikalfluss-Immunoassay (VFI) (100) und/oder einen Lateralfluss-Assay (LFA), die in die Vorratsbehältermembran (73) oder die Detektionsmembran (54) integriert sind, einschließlich einer Detektionsmembran (54), die eine VFI- oder LFA-Membran ist, die ein oder mehrere Biomarker-Detektionselemente (76) umfasst, wobei optional die Biomarker-Detektionselemente (76) Antikörper sind, wobei der Vertikalfluss-Immunoassay (100) Folgendes umfasst:
ein Sandwich-Enzym-Immunadsorptionsassay (ELISA) (101);
ein Mittel (56) zum Antreiben eines Fluidstroms durch die Detektionsmembran (54);
wobei die Vorrichtung dazu konfiguriert ist, das Vorhandensein des Biomarkers durch eine Änderung der optischen Farbe an der Membran zu reflektieren.

8. Kit nach einem der Ansprüche 1 bis 7, wobei der Rohreinsatz (50) Folgendes umfasst:
einen Einsatzkörper (150), der zum Einsetzen in einen Vorratsbehälter (71) des Sammelrohrs (40) geformt ist;
einen Flansch (151) an dem Einsatzkörper (150), der dazu konfiguriert ist, von einer Eingangswand (152) des Sammelrohrs (40) gestützt zu werden;
eine untere Oberfläche (58) mit einer zentralen Öffnung (59); und
wobei das Einstichende (60) einer Zacke (61) entspricht, die sich von der unteren Oberfläche (58) erstreckt, und das Einstichende (60) einer Vielzahl von physisch getrennten Zacken (61) entspricht.

## Revendications

1. Kit pour l'auto-collecte et le traitement d'un liquide biologique qui peut contenir un échantillon biologique ou un biomarqueur de l'échantillon biologique provenant d'un utilisateur comprenant :
un embout buccal (30) ;
un tube de collecte (40) raccordé fluidiquement à l'embout buccal (30) ;
un insert de tube (50) positionné dans le tube de collecte (40), ledit insert de tube (50) comprenant :
une extrémité de perçage (60) ;
un capuchon (70) conçu pour se raccorder au tube de collecte (40) et sceller fluidiquement le tube de collecte (40) par rapport à un environnement avoisinant, ledit capuchon (70) comprenant :
un réservoir intégré (71) conçu pour contenir un réactif biologique (72) conçu pour être utilisé avec l'échantillon biologique dans le tube de collecte (40) ; une membrane de réservoir (73) destinée à contenir le réactif biologique (72) dans le réservoir intégré (71) et à sceller temporairement le réservoir intégré (71) par rapport au tube de collecte (40), ladite extrémité de perçage (60) de l'insert de tube (50) étant conçue pour percer la membrane de réservoir (73) et entrer en contact fluidique avec le liquide biologique dans le tube de collecte (40) et le réactif biologique dans le réservoir intégré (71), et ladite membrane de réservoir (73) étant conçue pour être amovible après que le réactif biologique (72) a été introduit dans le liquide biologique dans le tube de collecte (40) ; et
une membrane de détection de biomarqueurs (54) conçue pour se raccorder au capuchon (70) après que la membrane de réservoir (73) a été retirée afin de détecter un ou plusieurs biomarqueurs dans le liquide biologique.

2. Kit selon la revendication 1, ledit insert de tube comprenant en outre :
un élément de positionnement (51) destiné à aligner et positionner l'extrémité de perçage (60) à une distance prédéfinie (52) de la membrane de réservoir (73).

3. Kit selon la revendication 1 ou 2, comprenant en outre :
la membrane de détection (54) positionnée au bas de l'insert de tube (50) ;
un moyen (56) destiné à entraîner un écoulement de fluide à travers la membrane de détection (54) afin d'obtenir une mesure biologique ; et
un dispositif de support (57) destiné à maintenir la membrane de détection (54).

4. Kit selon l'une quelconque des revendications 1 à 3, ladite membrane de réservoir (73) étant une membrane refermable.

5. Kit selon la revendication 4, ladite membrane refermable comprenant :
une couche adhésive interchangeable (74) ; et
un substrat poreux (75) comprenant des détecteurs de biomarqueurs (76).

6. Kit selon la revendication 5, lesdits détecteurs de biomarqueurs (76) étant choisis dans le groupe constitué par : les polynucléotides, les polypeptides, les anticorps, les acides nucléiques, les toxines, les bactéries, les virus, les vésicules biologiques, ledit dispositif comprenant en outre une pluralité de détecteurs de biomarqueurs uniques (76) agencés dans un microréseau (77) sur la membrane de détection (54) pour la détection multiplex de biomarqueurs dans un liquide biologique.

7. Kit selon l'une quelconque des revendications 1 à 6, comprenant en outre :
un dosage immunologique à flux vertical (VFI) (100) et/ou un dosage à flux latéral (LFA) intégré à la membrane de réservoir (73) ou à la membrane de détection (54), comprenant une membrane de détection (54) qui est une membrane de VFI ou de LFA comprenant un ou plusieurs éléments de détection de biomarqueurs (76), éventuellement les éléments de détection de biomarqueurs (76) sont des anticorps, ledit dosage immunologique à flux vertical (100) comprenant :
un dosage immuno-enzymatique (ELISA) en sandwich (101) ;
un moyen (56) destiné à entraîner un écoulement de fluide à travers la membrane de détection (54) ;
ledit dispositif étant conçu pour refléter la présence du biomarqueur par un changement de couleur optique au niveau de la membrane.

8. Kit selon l'une quelconque des revendications 1 à 7, ledit insert de tube (50) comprenant :
un corps d'insert (150) mis en forme de manière à être inséré dans un réservoir (71) du tube de collecte (40) ;
une bride (151) sur le corps d'insert (150) conçue pour être supportée par une paroi d'entrée (152) du tube de collecte (40) ;
une surface inférieure (58) comportant une ouverture centrale (59) ; et
ladite extrémité de perçage (60) correspondant à une pointe (61) s'étendant à partir de la surface inférieure (58) et ladite extrémité de perçage (60) correspondant à une pluralité de pointes physiquement séparées (61).
